# EUROPEAN PATENT APPLICATION

(11) **EP 4 674 359 A1**
(43) Date of publication of application: **07.01.2026**
(21) Application number: 24806612.8
(22) Date of filing: 15.05.2024
(51) Int. Cl.: A61B 10/00, G16H 80/00

(54) **MENSTRUAL PERIOD MANAGEMENT METHOD AND RELATED APPARATUS**

(30) Priority: 18.05.2023 CN 202310567347
(71) Applicant: Huawei Technologies Co., Ltd., Shenzhen, Guangdong 518129 (CN)
(72) Inventor: CHEN, Cheng, Shenzhen, Guangdong 518129 (CN); WANG, Lu, Shenzhen, Guangdong 518129 (CN); LI, Wanyu, Shenzhen, Guangdong 518129 (CN)
(74) Representative: Pfenning, Meinig & Partner mbB
(86) International application number: PCT/CN2024/093378
(87) International publication number: WO 2024/235262

(57) **Abstract**

This application discloses a menstrual period management method and a related apparatus. The method includes: when an X^{th} day after a first date is predicted as a first menstrual day of a first menstrual period, displaying first prompt information and a first correction control, where the first prompt information is used to remind a user that the menstrual period arrives within X days, and the first correction control indicates that the first menstrual day of the first menstrual period has arrived, and is further used to correct the first menstrual day of the first menstrual period; and if a first input operation is detected, correcting and calibrating the first menstrual day of the first menstrual period to a second date, where the first input operation includes a second input operation acting on the first correction control, the second date is earlier than or equal to the first date, and the calibrated first menstrual day is used to predict a future menstrual period. In this way, a user operation is simple, timely and accurate menstrual period management is implemented, and user experience is effectively improved.

## Description

This application claims priority to Chinese Patent Application No. 202310567347.3, filed with the China National Intellectual Property Administration on May 18, 2023 and entitled "MENSTRUAL PERIOD MANAGEMENT METHOD AND RELATED APPARATUS", which is incorporated herein by reference in its entirety.

### TECHNICAL FIELD

This application relates to the field of electronic technologies, and in particular, to a menstrual period management method and a related apparatus.

### BACKGROUND

Women pay more attention to health. Menstruation is a physiological phenomenon of women of childbearing age. A menstrual period (that is, a period) and a menstrual cycle (that is, a physiological cycle) can indicate a physical health status of a woman of childbearing age. Studies show that adjusting training and nutrition plans in combination with a physiological period and a physiological cycle can fully benefit the body and the mind. Menstrual period management has become a core foundation of other services such as women's health management, pregnancy preparation management, and sex life.

Currently, a user may perform menstrual period management by using an application installed on a mobile phone. However, an existing menstrual period management manner usually depends on an input of the user, including a feedback of data such as start time and end time of a historical menstrual period. For the user, operations are complex, and the user may forget to provide an input. The existing menstrual period management manner cannot perform timely and accurate menstrual period management, and user experience is poor.

### SUMMARY

This application provides a menstrual period management method and a related apparatus, so that a user operation is simple, timely and accurate menstrual period management is implemented, and user experience is effectively improved.

According to a first aspect, this application provides a menstrual period management method, applied to a first electronic device, and including: when an X^{th} day after a first date is predicted as a first menstrual day of a first menstrual period, displaying first prompt information and a first correction control, where the first prompt information is used to remind a user that the menstrual period arrives within X days, and the first correction control indicates that the first menstrual day of the first menstrual period has arrived, and is further used to correct the first menstrual day of the first menstrual period; and if a first input operation is detected, correcting and calibrating the first menstrual day of the first menstrual period to a second date, where the first input operation includes a second input operation acting on the first correction control, the second date is earlier than or equal to the first date, and the calibrated first menstrual day is used to predict a future menstrual period.

Through implementation of embodiments of this application, based on advance prediction and reminding that are X days before a menstrual period, the user can be reminded in time that the menstrual period is to arrive, so that the user performs preparation in advance. When an actual menstrual period has arrived, the user is prompted, by using a correction control, to manually calibrate a first menstrual day. In this way, a user operation is simple, timely and accurate menstrual period management is implemented, and user experience is effectively improved.

In an implementation, a date selection control is displayed in response to the second input operation acting on the first correction control, where the date selection control is configured to select a corrected date, the date selection control includes a date option of the second date, and the first input operation further includes a third input operation acting on the date option of the second date. Through implementation of embodiments of this application, when the actual menstrual period has arrived, the user may select a date option of an actual first menstrual day based on the actual first menstrual day.

In an implementation, a first confirmation control is displayed after the third input operation acting on the date option of the second date is detected, and the first input operation further includes a fourth input operation acting on the first confirmation control.

In an implementation, when it is detected that a third date is the first menstrual day, the third date is calibrated as the first menstrual day of the first menstrual period, and second prompt information and a second correction control are displayed, where the second prompt information indicates that the third date is the first menstrual day, and the second correction control is used to correct the first menstrual day of the first menstrual period. Through implementation of embodiments of this application, when the first menstrual day is detected, the user is prompted in time, so that when the first menstrual day is incorrect, the actual first menstrual day is calibrated in time by using the correction control. In this way, a user operation is simple, accuracy of the calibrated first menstrual day is improved, and user experience is effectively improved.

In an implementation, after the second prompt information and the second correction control are displayed, the method further includes: if a fifth input operation is detected, correcting and calibrating the first menstrual day of the first menstrual period to a fourth date, where the fifth input operation includes a sixth input operation acting on the second correction control.

In an implementation, the method further includes: displaying a date option of the fourth date in response to the sixth input operation, where the fifth input operation further includes an operation acting on the date option of the fourth date.

In an implementation, the method further includes: further displaying a first cancellation option in response to the sixth input operation, where the first cancellation option indicates that the first menstrual day of the first menstrual period has not arrived; and if a seventh input operation acting on the first cancellation option is detected, canceling calibrating the third date as the first menstrual day of the first menstrual period. Through implementation of embodiments of this application, when the actual menstrual period has not arrived, the user may cancel the preliminarily calibrated first menstrual day, to improve accuracy of the first menstrual day in time.

In an implementation, the method further includes: further displaying a first cancellation control along with the second prompt information, where the first cancellation control indicates that the first menstrual day of the first menstrual period has not arrived, and is used to cancel calibrating the third date as the first menstrual day of the first menstrual period. Through implementation of embodiments of this application, when the actual menstrual period has not arrived, the user may cancel the preliminarily calibrated first menstrual day, to improve accuracy of the first menstrual day in time.

In an implementation, the method further includes: when it is detected that a fifth date is a last menstrual day, calibrating the fifth date as the last menstrual day of the first menstrual period, and displaying third prompt information and a third correction control, where the third prompt information indicates that the fifth date is the last menstrual day of the first menstrual period, the third correction control is used to correct the last menstrual day of the first menstrual period, and the calibrated last menstrual day is used to predict the future menstrual period. Through implementation of embodiments of this application, when the last menstrual day is detected, the user is prompted in time, so that when the last menstrual day is incorrect, the actual last menstrual day is manually calibrated in time by using the correction control. In this way, a user operation is simple, accuracy of the calibrated last menstrual day is improved, and user experience is effectively improved.

In an implementation, after the displaying third prompt information and a third correction control, the method further includes: if an eighth input operation is detected, correcting and calibrating the last menstrual day of the first menstrual period to a sixth date, where the eighth input operation includes a ninth input operation acting on the third correction control.

In an implementation, the method further includes: displaying a date option of the sixth date in response to the ninth input operation, where the eighth input operation further includes an operation acting on the date option of the sixth date.

In an implementation, the method further includes: further displaying a second cancellation option in response to the ninth input operation, where the second cancellation option indicates that the first menstrual period has not ended; and if a tenth input operation acting on the second cancellation option is detected, canceling calibrating the fifth date as the last menstrual day of the first menstrual period. Through implementation of embodiments of this application, when the actual menstrual period has not ended, the user may cancel the preliminarily calibrated last menstrual day, to improve accuracy of the last menstrual day in time.

In an implementation, the method further includes: further displaying a second cancellation control along with the third prompt information, where the second cancellation control indicates that the first menstrual period has not ended, and is used to cancel calibrating the fifth date as the last menstrual day of the first menstrual period. Through implementation of embodiments of this application, when the actual menstrual period has not ended, the user may cancel the preliminarily calibrated last menstrual day, to improve accuracy of the last menstrual day in time.

In an implementation, the first electronic device is further configured to obtain K types of physiological features of the user on every day based on data collected by a detection apparatus, and the method further includes: detecting a menstrual period trend value of a current date based on K types of physiological features of the user on last N days, where the menstrual period trend value of the current date indicates a degree to which the current date is close to a peak of a menstrual period of the user, N and K are positive integers, and the menstrual period trend value is used to predict and calibrate a first menstrual day and a last menstrual day. In this way, compared with a conventional menstrual period management method, this method does not strongly depend on a user input of menstrual period information, and the menstrual period trend value is measured by using a detected physiological sign of the user, to intelligently calibrate a menstrual period interval, thereby reducing a long-term recording burden of the user.

In an implementation, the method further includes: updating and displaying a menstrual period trend curve and a first indication box based on the menstrual period trend value of the current date, where the menstrual period trend curve sequentially indicates a detected menstrual period trend value of a date before the current date, the menstrual period trend value of the current date, and a predicted menstrual period trend value of a date after the current date, the first indication box indicates a menstrual period trend value on every day within a menstrual period range on the menstrual period trend curve, and the menstrual period range is a time range from the first menstrual day to the last menstrual day. In this way, based on the foregoing menstrual period trend curve, the user obtains a dynamical change of a menstrual period trend, and learns of a physiological cycle feature of the user.

In an implementation, a menstrual period trend curve corresponding to a date after the current date in the menstrual period trend curve is determined based on a historical menstrual period trend curve/historical menstrual period trend sequence obtained through comprehensive statistics collection; the historical menstrual period trend sequence is determined through comprehensive statistics collection based on a detected menstrual period trend value in at least one historical physiological cycle; the historical menstrual period trend sequence indicates an average length of historical physiological cycles obtained through comprehensive statistics collection and a menstrual period trend value on every day in one historical physiological cycle obtained through comprehensive statistics collection; the historical menstrual period trend curve is determined based on the historical menstrual period trend sequence; and the historical menstrual period trend sequence further indicates a menstrual period range in one physiological cycle corresponding to the historical menstrual period trend sequence, and the menstrual period range indicated by the historical menstrual period trend sequence is determined based on a menstrual period calibrated in the at least one historical physiological cycle.

In an implementation, the method further includes: displaying a first indicator on the menstrual period trend curve, where the first indicator indicates a point corresponding to the current date on the menstrual period trend curve; and after an eleventh input operation acting on the first indicator is detected, displaying a first interface, where the first interface is used to edit and display a physical symptom corresponding to the current date. In this way, the user intuitively senses a physical symptom in each period of a physiological cycle, to better cope with the physical symptom subsequently.

In an implementation, the K types of physiological features include a first physiological feature, and the method further includes: further displaying one or more of the following physiological feature curves of the first physiological feature along with the menstrual period trend curve: a measured physiological feature curve, a historical minimum physiological feature curve, a historical average physiological feature curve, and a historical average physiological feature curve, where the menstrual period trend curve has a same horizontal coordinate as each physiological feature curve, and the measured physiological feature curve and each historical physiological feature curve are aligned in time domain by using a preset algorithm; the measured physiological feature curve indicates physiological feature values measured on the current date and a previous date; the historical minimum physiological feature curve indicates a minimum physiological feature value on every day in one historical physiological cycle obtained through comprehensive statistics collection; the historical average physiological feature curve indicates an average physiological feature value on every day in one historical physiological cycle obtained through comprehensive statistics collection; and the historical maximum physiological feature curve indicates a maximum physiological feature value on every day in one historical physiological cycle obtained through comprehensive statistics collection. In this way, the user can more dynamically sense a fluctuation feature of a menstrual period, and learn of a physiological cycle feature of the user in a multi-dimensional manner.

In an implementation, the method further includes: detecting, based on a detected menstrual period trend value of the user, whether an abnormal situation exists; and displaying fourth prompt information when an abnormal situation is detected, where the fourth prompt information indicates the abnormal situation.

In an implementation, the abnormal situation includes one or more of the following: the user is changed; the user is not a female; the user is sick; or the user is pregnant; and when the abnormal situation is that the user is not a female, the fourth prompt information is further used to prompt the user to disable a related function for a menstrual period; or when the abnormal situation is that the user is pregnant, the fourth prompt information is further used to prompt the user to switch to a pregnancy mode.

In an implementation, the method further includes: on an F^{th} day after a last menstrual day of the first menstrual period, re-calibrating the first menstrual day and the last menstrual day of the first menstrual period based on detected menstrual period trend values on last C days and a historical menstrual period trend sequence, where C is greater than F, and both C and F are positive integers.

In an implementation, the re-calibrating the first menstrual day and the last menstrual day of the first menstrual period includes: when a first condition is detected, re-calibrating the first menstrual day and the last menstrual day of the first menstrual period, where the first condition includes one or more of the following: the first menstrual day and/or the last menstrual day of the first menstrual period are/is not calibrated; the first menstrual day and/or the last menstrual day are/is calibrated, but the user does not provide a feedback for the calibrated first menstrual day and/or last menstrual day; or the calibrated first menstrual day and/or last menstrual day are/is clearly incorrect. Intelligent re-calibration based on a menstrual period trend value present after a menstrual period ends can effectively reduce an error of a calibrated menstrual period, so as to improve accuracy of subsequent menstrual period prediction, and effectively cope with various situations that may cause a large menstrual period error.

In an implementation, when the menstrual period trend value of the current date meets a second condition, an X^{th} day after the current date is predicted as the first menstrual day of the first menstrual period, where the second condition includes: a difference between the menstrual period trend value of the current date and a first threshold is less than a preset value, and the first threshold is a menstrual period trend value of an X^{th} day before the first menstrual day that is determined based on a historical menstrual period trend sequence.

In an implementation, when the menstrual period trend value of the current date meets a third condition, the current date is predicted as the first menstrual day of the first menstrual period, where the second condition includes: on the current date and a previous date, the menstrual period trend value continuously increases by w days, and the menstrual period trend value of the current date is greater than a first-day threshold for the first time within the w days.

In an implementation, when the menstrual period trend value of the current date meets a fourth condition, the current date is predicted as a last menstrual day of the first menstrual period, where the fourth condition includes: on the current date and a previous date, the menstrual period trend value continuously increases by w days and then continuously decreases by u days, and the menstrual period trend value of the current date is less than a last-day threshold for the first time within the u days.

According to a second aspect, this application provides an electronic device, including one or more processors and one or more memories. The one or more memories are coupled to the one or more processors. The one or more memories are configured to store computer program code, and the computer program code includes computer instructions. When the one or more processors execute the computer instructions, the electronic device is enabled to perform the menstrual period management method according to any one of the possible implementations of any one of the foregoing aspects.

According to a third aspect, an embodiment of this application provides a computer storage medium, including computer instructions. When the computer instructions are run on an electronic device, the electronic device is enabled to perform the menstrual period management method according to any one of the possible implementations of any one of the foregoing aspects.

According to a fourth aspect, an embodiment of this application provides a computer program product. When the computer program product runs on a computer, the computer is enabled to perform the menstrual period management method according to any one of the possible implementations of any one of the foregoing aspects.

### BRIEF DESCRIPTION OF DRAWINGS

FIG. 1 is a diagram of a system architecture of a communication system according to an embodiment of this application;
FIG. 2 is a diagram of a structure of an electronic device according to an embodiment of this application;
FIG. 3A to FIG. 3H show related user interfaces of a Health app according to an embodiment of this application;
FIG. 4A to FIG. 4C show related user interfaces of advance prediction and reminding of a smart band according to an embodiment of this application;
FIG. 5A to FIG. 5E show related user interfaces of first menstrual day reminding of a smart band according to an embodiment of this application;
FIG. 6A to FIG. 6E show related user interfaces of last menstrual day reminding of a smart band according to an embodiment of this application;

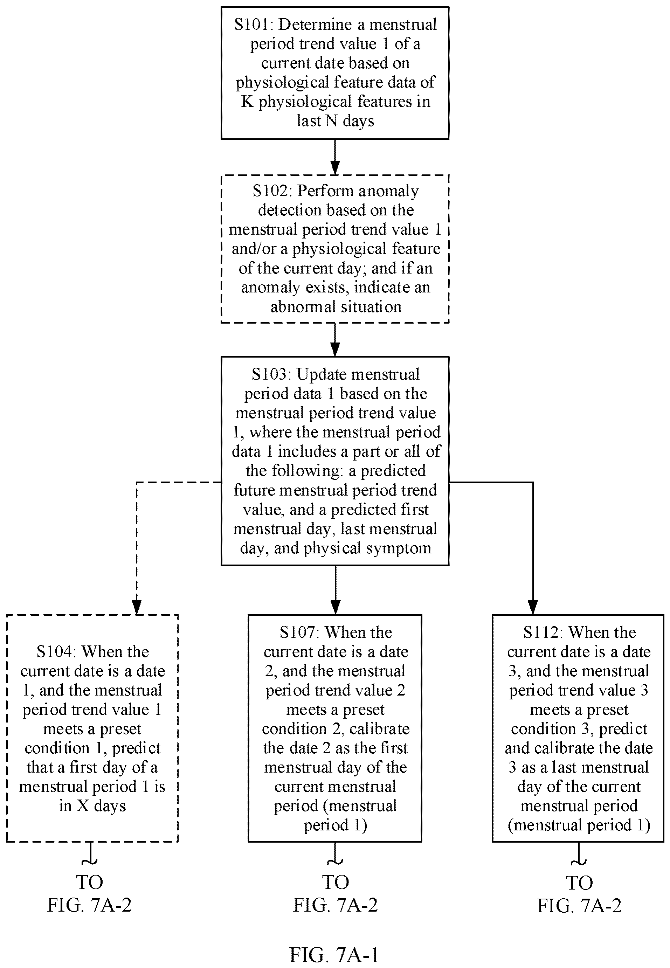

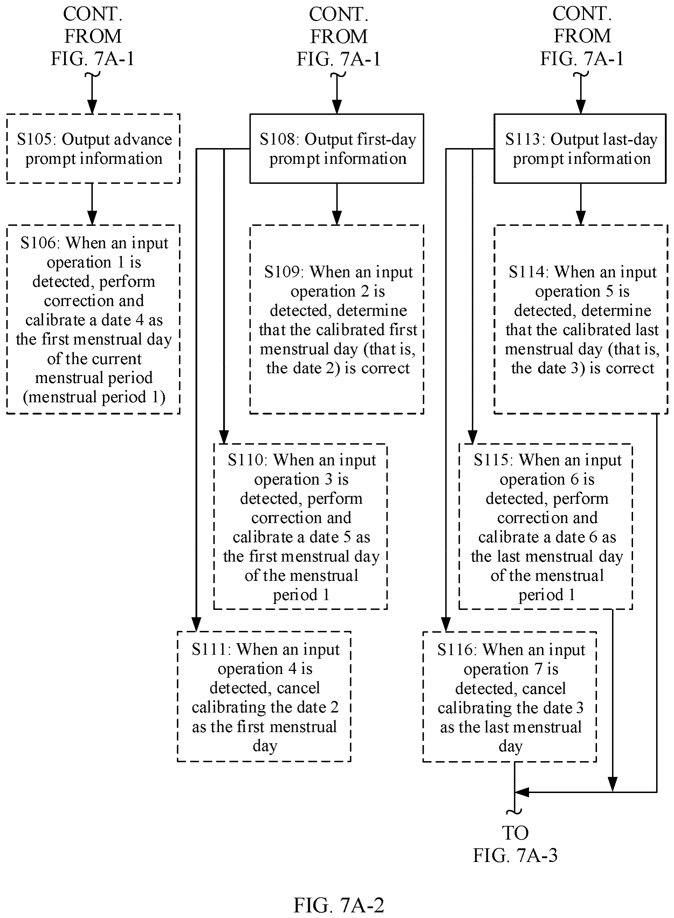

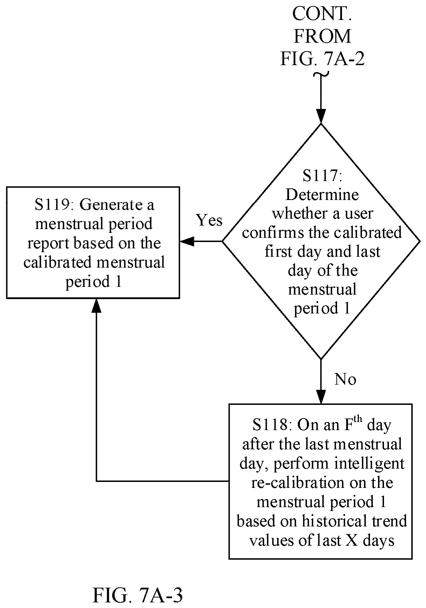

FIG. 7A-1 to FIG. 7A-3 show a method procedure of a menstrual period management method according to an embodiment of this application;

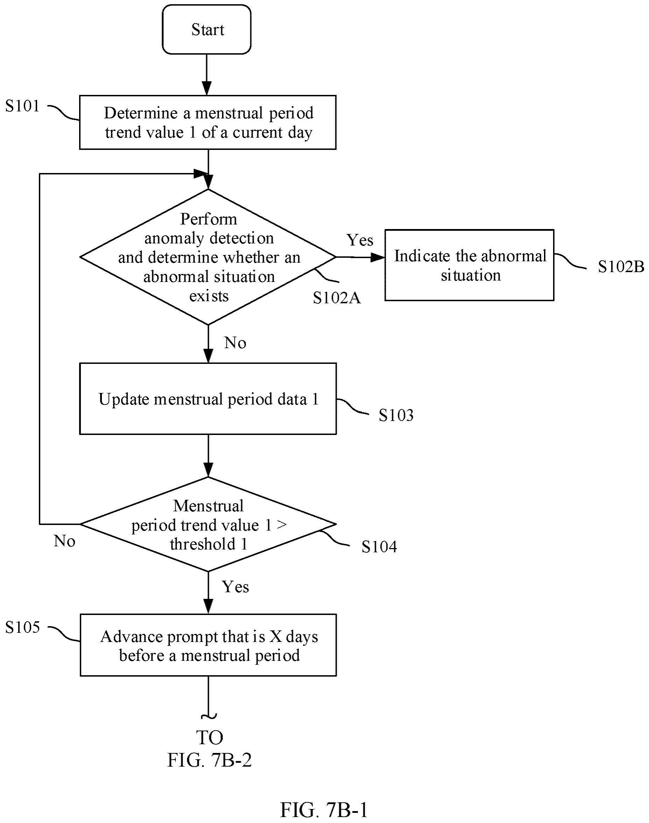

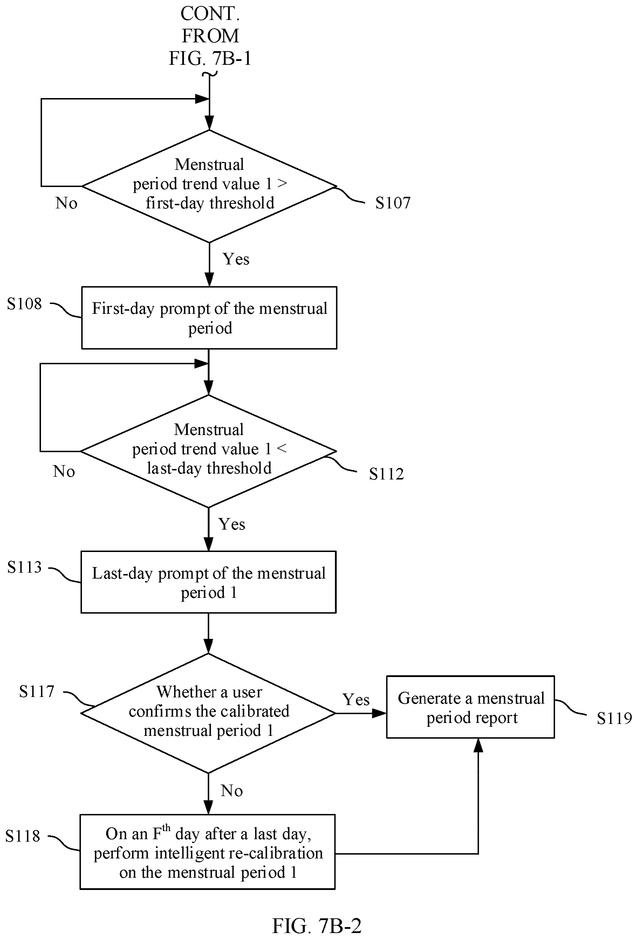

FIG. 7B-1 and FIG. 7B-2 show a method procedure of a menstrual period management method according to an embodiment of this application;

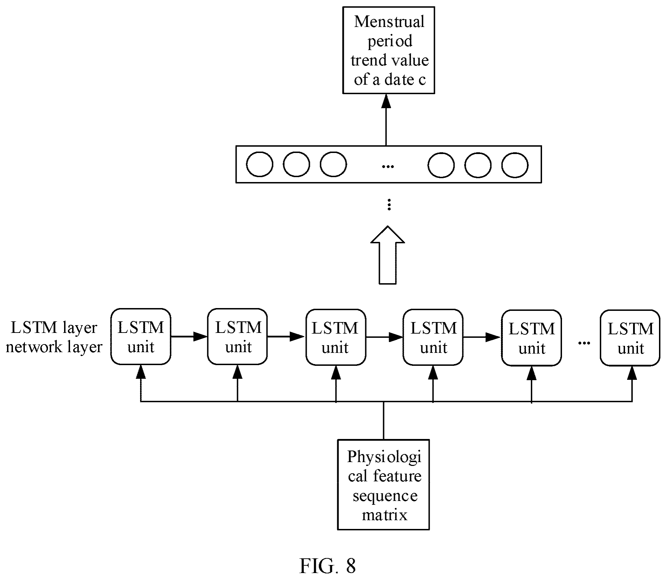

FIG. 8 shows an LSTM-based learning model according to an embodiment of this application;

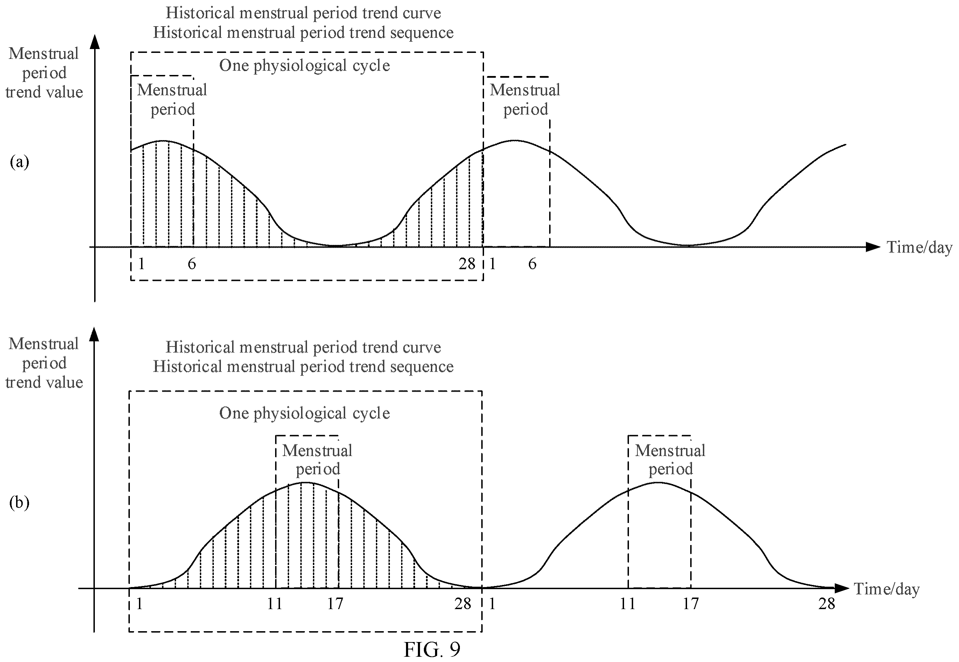

FIG. 9 is a diagram of a historical menstrual period trend curve according to an embodiment of this application;

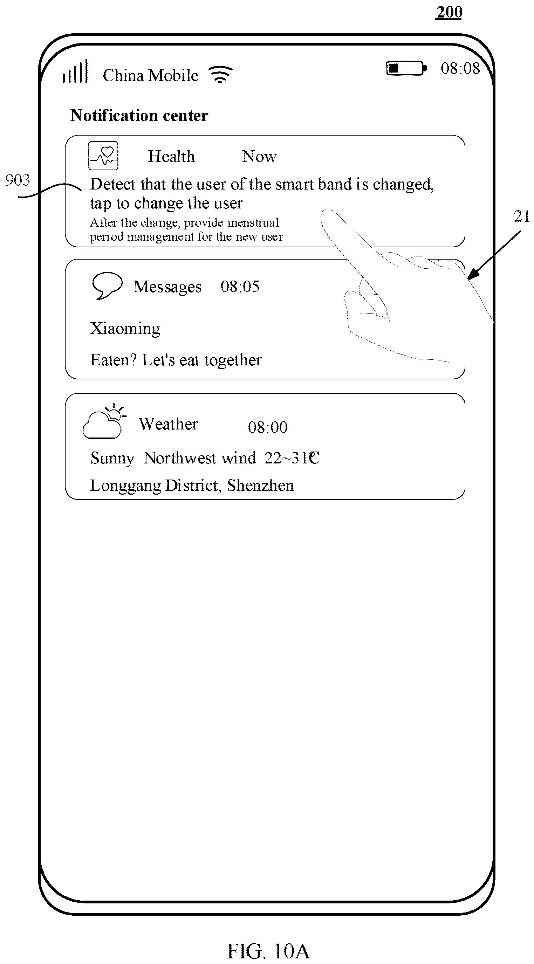

FIG. 10A to FIG. 10D show related user interfaces of user switching according to an embodiment of this application;

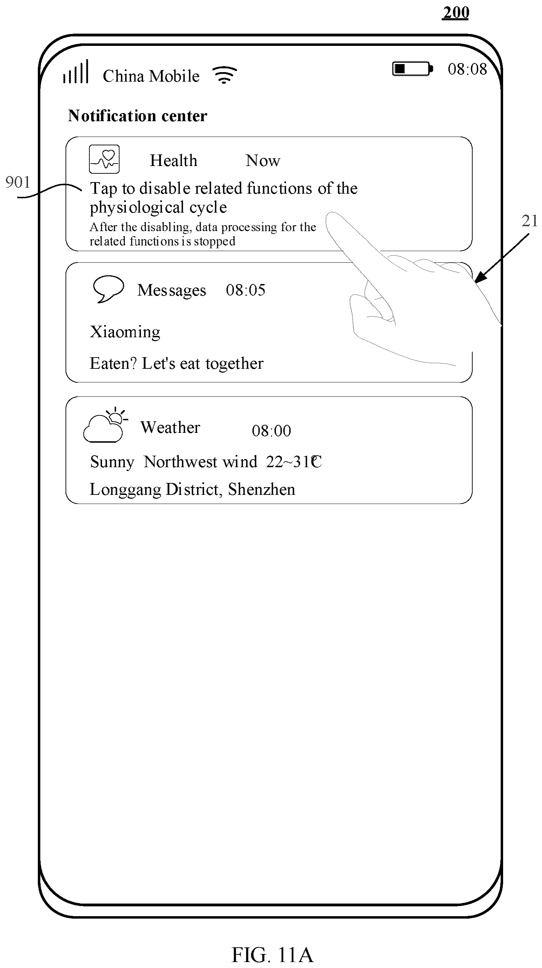

FIG. 11A to FIG. 11C show related user interfaces of disabling a menstrual period management function according to an embodiment of this application;

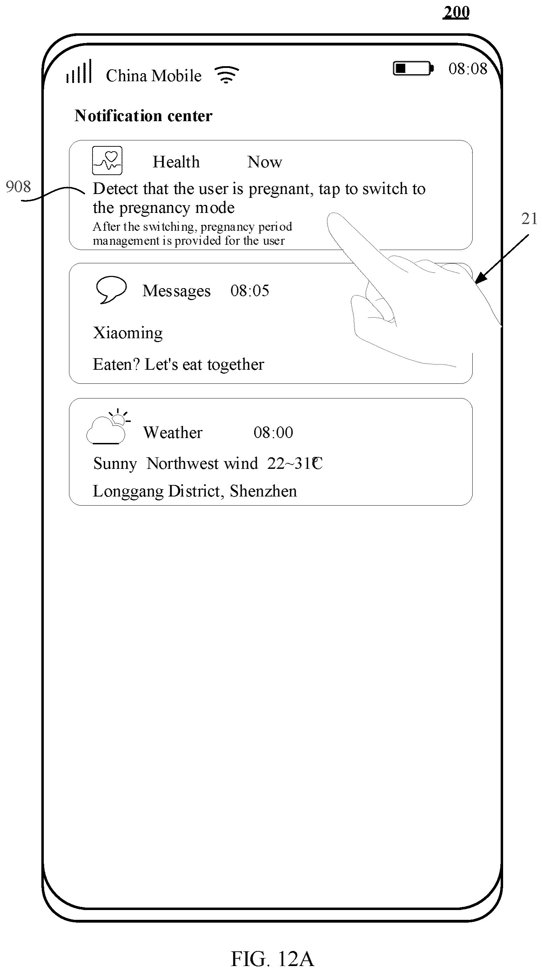

FIG. 12A to FIG. 12C show related user interfaces of switching to a pregnancy mode according to an embodiment of this application; and

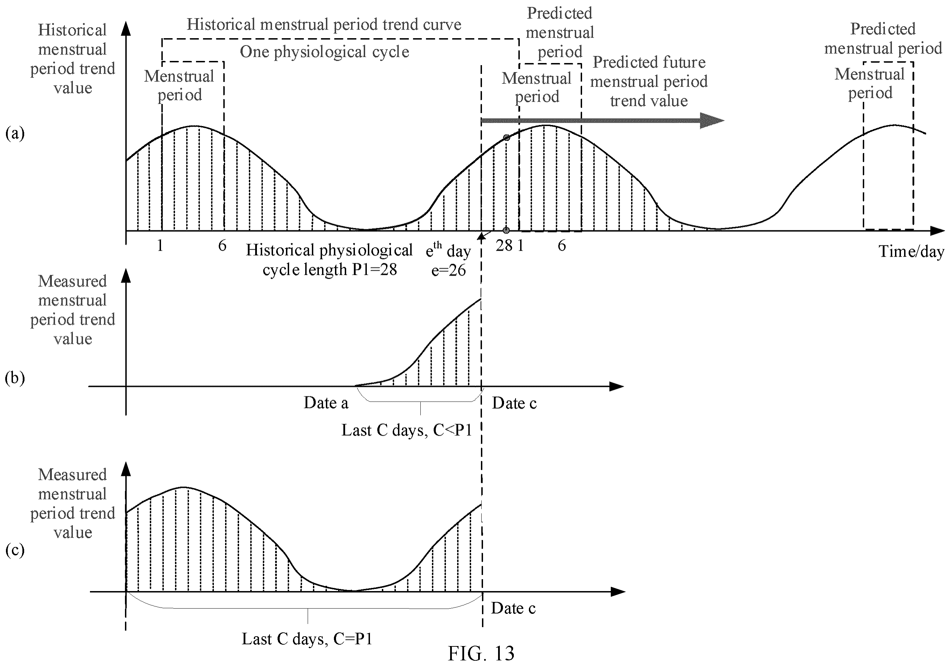

FIG. 13 is a diagram of a menstrual period trend sequence according to an embodiment of this application.

### DESCRIPTION OF EMBODIMENTS

Technical solutions in embodiments of this application are clearly described below in detail with reference to the accompanying drawings. In the descriptions of embodiments of this application, unless otherwise specified, "/" indicates "or". For example, A/B may indicate A or B. The term "and/or" in this specification merely describes an association relationship for describing associated objects, and indicates that three relationships may exist. For example, A and/or B may indicate the following three cases: Only A exists, both A and B exist, and only B exists. In addition, in the descriptions of embodiments of this application, "a plurality of" means two or more.

The following terms "first" and "second" are merely used for description, but should not be understood as indicating or implying relative importance or implying a quantity of indicated technical features. Therefore, a feature limited by "first" or "second" may explicitly or implicitly include one or more such features. In the descriptions of embodiments of this application, unless otherwise specified, "a plurality of" means two or more.

The term "user interface (user interface, UI)" in the following embodiments of this application is a medium interface for interaction and information exchange between an application or an operating system and a user, and implement conversion between an internal form of information and an acceptable form of the user. A user interface is source code compiled in a specific computer language such as Java or the extensible markup language (extensible markup language, XML). The interface source code is parsed and rendered on an electronic device, and finally presented as content that can be recognized by the user. A common presentation form of the user interface is a graphic user interface (graphic user interface, GUI), which refers to a user interface that is displayed in a graphical manner and that is related to a computer operation. The GUI may be a visible interface element displayed on a display of an electronic device, for example, a text, an icon, a button, a menu, a tab, a text box, a dialog box, a status bar, a navigation bar, or a widget.

In embodiments of this application, a menstrual period includes a phase/time period in which a menstrual phenomenon occurs in a uterus in a menstrual cycle, and may also be referred to as a period or a physiological period. A length of the menstrual period is usually about seven days, for example, six days. A menstrual cycle is a cycle in which a menstrual phenomenon occurs, and may also be referred to as a physiological cycle. A length of the physiological cycle is usually about 28 days, for example, 28 days or 29 days. According to a physiological feature of a user, a physiological cycle may usually include phases of a plurality of physiological phenomena, including a menstrual period, a follicular period, an ovulatory period, a luteal period, a fertile period, a safe period, and the like. Different users may have different menstrual period lengths and physiological cycle lengths, and a menstrual period length and a physiological cycle length of a same user may also change.

A current menstrual period management method mainly depends on a conventional calendar method. The method is applied to menstrual period management applications (Application, APP) installed on most mobile phones currently. Specifically, the method mainly depends on an active input of a user, including: The user needs to actively tap a date in a calendar to enter a date of a first day and a date of a last day of a menstrual period, and the user needs to manually enter a menstrual period length (for example, six days) and a physiological cycle length (for example, 28 days). In the method, it is assumed that the menstrual period of the user does not fluctuate, and has a relatively fixed menstrual period length and physiological cycle length, and a future menstrual period is determined by using only information entered by the user. If the user does not actively enter information, a length of the determined future menstrual period is fixed, and fluctuation of the menstrual period, for example, a delayed or advanced menstrual period, a menstrual period length change, or a physiological cycle length change, is not considered. It may be understood that changes in an emotion, an exercise status, a health status, and an environment of the user may cause fluctuation of the menstrual period. If the user forgets to feed back menstrual information, an error of the calibrated menstrual period is increasingly larger after no feedback is provided in a plurality of cycles.

This application provides a menstrual period management method, to implement more accurate menstrual period management, including menstrual period prediction, reminding, and correction, thereby effectively improving user experience. The following describes a communication system 10 related to the menstrual period management method provided in embodiments of this application.

The communication system 10 includes one or more detection apparatuses configured to collect a physiological feature of a user. The detection apparatus may be a wearable device (for example, a heart rate band, a smart band, or an electronic body temperature patch). A wearing location and a type of the wearable device are not specifically limited in embodiments of this application. In some embodiments, the communication system 10 includes only one detection apparatus, namely, the foregoing wearable device (for example, a smart band).

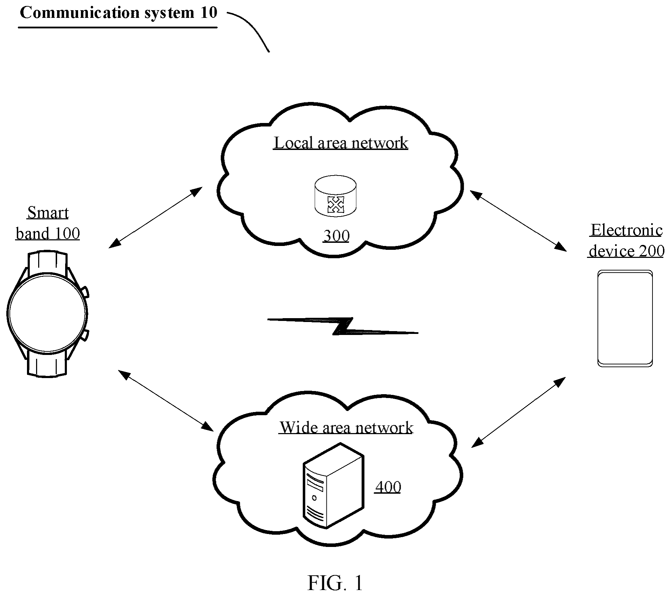

In some embodiments, as shown in FIG. 1, one detection apparatus (for example, a smart band 100) in the communication system 10 has a display. When there is another detection apparatus, the smart band 100 may establish a communication connection to the another detection apparatus. The smart band 100 may obtain a physiological feature collected by each detection apparatus; and perform menstrual period prediction, reminding, and correction based on the detected physiological feature, and display related information of a menstrual period, for example, some or all of the following: advance prediction and prompt that are X days before the menstrual period, a first menstrual day prompt, a control for correcting a first menstrual day, a last menstrual day prompt, a control for correcting a last menstrual day, a menstrual period trend curve, a predicted menstrual period, the detected physiological feature, and a menstrual period report.

In some embodiments, the communication system 10 further includes an electronic device 200 (for example, a mobile phone). The electronic device 200 is configured with a display. The electronic device 200 may establish a communication connection to the foregoing one or more detection apparatuses, to obtain the physiological feature collected by each detection apparatus; and perform menstrual period prediction, reminding, and correction based on the detected physiological feature, and display the related information of the menstrual period.

In some embodiments, the communication system 10 includes the electronic device 200 and the smart band 100. The electronic device 200 establishes a communication connection to the smart band 100. One of the electronic device 200 and the smart band 100 may establish a communication connection to the foregoing one or more detection apparatuses, to obtain the physiological feature collected by each detection apparatus, and perform menstrual period prediction and correction based on the detected physiological feature. The electronic device 200 and/or the smart band 100 may perform menstrual period reminding, and display the related information of the menstrual period.

In some embodiments, two devices (for example, the electronic device 200 and the smart band 100) in the communication system 10 may be directly connected by using a local wired connection. In some embodiments, the foregoing two devices may be directly connected by using a short-range wireless communication module. For example, the electronic device 200 and the smart band 100 may have one or more of short-range communication modules such as a near field communication (near field communication, NFC) communication module, a wireless fidelity (wireless fidelity, Wi-Fi) communication module, an ultra-wideband (ultra-wideband, UWB) communication module, a Bluetooth (Bluetooth) communication module, and a ZigBee communication module.

In some embodiments, the electronic device 200 and the smart band 100 may be connected to a local area network (local area network, LAN) through an electronic device 300 based on a wired connection or a Wi-Fi connection. For example, the electronic device 300 may be a third-party device such as a router, a gateway, or a smart device controller. In some embodiments, the electronic device 200 and the smart band 100 may alternatively be indirectly connected through at least one electronic device 400 in a wide area network. The electronic device 400 may be a hardware server, or may be a cloud server embedded in a virtualized environment. It may be understood that the electronic device 300 and/or the electronic device 400, the electronic device 200, and the smart band 100 indirectly perform wireless communication connection and data transmission.

In an application scenario, an app (that is, an app 1) used for menstrual period management is installed on the electronic device 200 and the smart band 100. The electronic device 200 and the smart band 100 log in to a same account by using a server of the app, to establish an indirect connection. In some embodiments, one of the electronic device 200 and the smart band 100 may establish a communication connection to the foregoing one or more detection apparatuses, to obtain the physiological feature collected by each detection apparatus, and upload the physiological feature to the server. Then, the server performs menstrual period prediction and intelligently re-calibrates a calibrated menstrual period based on the detected physiological feature. The server indicates the electronic device 200 and/or the smart band 100 to perform menstrual period reminding and display the related information of the menstrual period.

It may be understood that the structure shown in this embodiment does not constitute a specific limitation on the communication system 10. In some other embodiments of this application, the communication system 10 may include more or fewer devices than those shown in the figure.

A type of the electronic device 200 is not specifically limited in embodiments of this application. For example, the electronic device 200 in embodiments of this application may be a device such as a mobile phone, a wearable device (for example, a smart band or a smartwatch), a tablet computer, a laptop computer (laptop), a handheld computer, a notebook computer, an invehicle device, a smart television, an ultra-mobile personal computer (ultra-mobile personal computer, UMPC), a cellular phone, a personal digital assistant (personal digital assistant, PDA), or an augmented reality (Augmented reality, AR)\virtual reality (virtual reality, VR) device. The electronic device 200 and the smart band may have a same operating system/different operating systems, for example, iOS, Android, Microsoft, or another operating system.

The following describes a structure of the electronic device 200 provided in an embodiment of this application. For a structure of the wearable device (for example, the smart band 100) in embodiments of this application, refer to related descriptions of the electronic device 200. Details are not described subsequently.

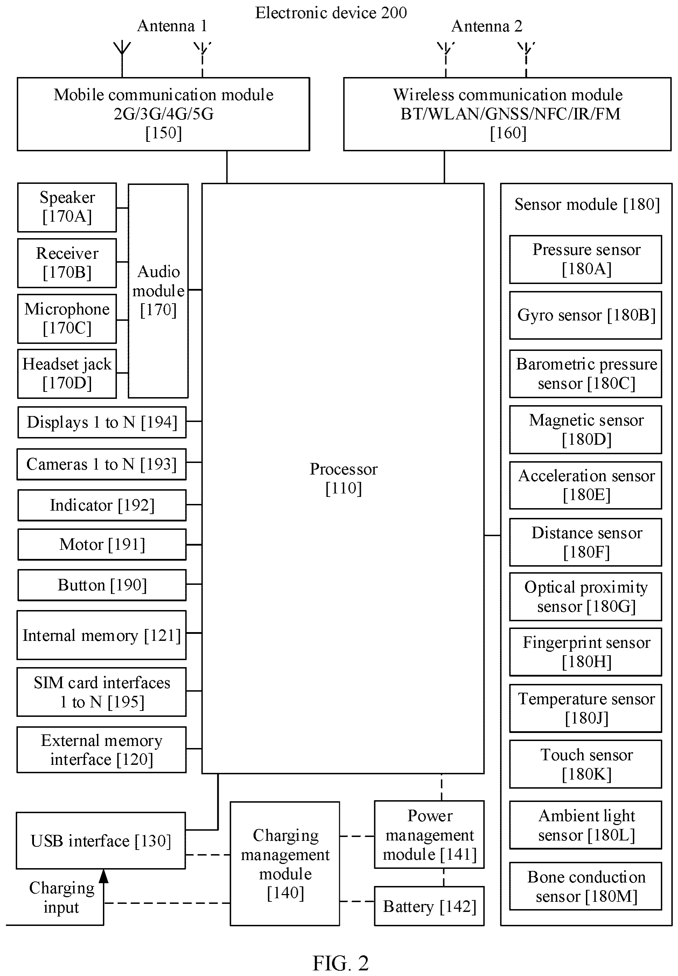

For example, as shown in FIG. 2, the electronic device 200 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 200. In some other embodiments of this application, the electronic device 200 may include more or fewer components than those shown in the figure, or combine some components, or split some components, or have different component arrangements. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on instruction operation code and a time sequence signal, to complete control of instruction fetching and instruction execution.

A memory may be further disposed in the processor 110, to store instructions and data. In some embodiments, the memory in the processor 110 is a cache. The memory may store instructions or data just used or cyclically used by the processor 110. If the processor 110 needs to use the instructions or data again, the instructions or data may be directly invoked from the memory. This avoids repeated access, and reduces waiting time of the processor 110, so that system efficiency is improved.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronous serial bus, and includes a serial data line (serial data line, SDA) and a serial clock line (derail clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K by using an I2C interface, so that the processor 110 communicates with the touch sensor 180K by using the I2C bus interface, to implement a touch function of the electronic device 200.

The I2S interface may be used for audio communication. In some embodiments, the processor 110 may include a plurality of groups of I2S buses. The processor 110 may be coupled to the audio module 170 by using the I2S bus, to implement communication between the processor 110 and the audio module 170. In some embodiments, the audio module 170 may transmit an audio signal to the wireless communication module 160 through the I2S interface, to implement a function of answering a call by using a Bluetooth headset.

The PCM interface may be configured to perform audio communication, and sample, quantize, and code analog signals. In some embodiments, the audio module 170 may be coupled to the wireless communication module 160 by using a PCM bus interface. In some embodiments, the audio module 170 may also transmit an audio signal to the wireless communication module 160 through the PCM interface, to implement a function of answering a call by using a Bluetooth headset. Both the I2S interface and the PCM interface may be used for audio communication.

The UART interface is a universal serial data bus, and is used for asynchronous communication. The bus may be a two-way communication bus. The bus converts to-betransmitted data between serial communication and parallel communication. In some embodiments, the UART interface is usually configured to connect the processor 110 to the wireless communication module 160. For example, the processor 110 communicates with a Bluetooth module in the wireless communication module 160 by using the UART interface, to implement a Bluetooth function. In some embodiments, the audio module 170 may transmit an audio signal to the wireless communication module 160 through the UART interface, to implement a function of playing music by using a Bluetooth headset.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 and the camera 193 communicate with each other by using the CSI interface, to implement a photographing function of the electronic device 200. The processor 110 communicates with the display 194 by using the DSI interface, to implement a display function of the electronic device 200.

The GPIO interface may be configured by using software. The GPIO interface may be configured as a control signal, or may be configured as a data signal. In some embodiments, the GPIO interface may be configured to connect the processor 110 to the camera 193, the display 194, the wireless communication module 160, the audio module 170, the sensor module 180, and the like. The GPIO interface may alternatively be configured as the I2C interface, the I2S interface, the UART interface, the MIPI interface, or the like.

The USB interface 130 is an interface conforming to a USB standard specification, and may be specifically a mini USB interface, a micro USB interface, a USB Type-C interface, or the like. The USB interface 130 may be configured to connect to a charger to charge the electronic device 200, or may be configured to transmit data between the electronic device 200 and a peripheral device, or may be configured to connect to a headset to play audio by using the headset. The interface may alternatively be configured to connect to another electronic device, such as an AR device.

It may be understood that the interface connection relationship between the modules shown in this embodiment of the present invention is merely an example, and does not constitute a limitation on the structure of the electronic device 200. In some other embodiments of this application, the electronic device 200 may alternatively use an interface connection manner different from that in the foregoing embodiment, or a combination of a plurality of interface connection manners.

The charging management module 140 is configured to receive charging input from a charger. The charger may be a wireless charger, or may be a wired charger. In some wired charging embodiments, the charging management module 140 may receive charging input from a wired charger through the USB interface 130. In some wireless charging embodiments, the charging management module 140 may receive wireless charging input through a wireless charging coil of the electronic device 200. When charging the battery 142, the charging management module 140 may further supply power to the electronic device through the power management module 141.

The power management module 141 is configured to connect the battery 142, the charging management module 140, and the processor 110. The power management module 141 receives input of the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device 200 may be implemented by using the antenna 1, the antenna 2, the mobile communication module 150, the wireless communication module 160, the modem processor, the baseband processor, and the like.

The antenna 1 and the antenna 2 are configured to transmit and receive electromagnetic wave signals. Each antenna in the electronic device 200 may be configured to cover one or more communication bands. Different antennas may be multiplexed to improve antenna utilization. For example, the antenna 1 may be multiplexed into a diversity antenna of a wireless local area network. In some other embodiments, the antenna may be used in combination with a tuning switch.

The mobile communication module 150 may provide a solution for wireless communication, including 2G/3G/4G/5G and the like, that is applied to the electronic device 200. The mobile communication module 150 may include at least one filter, a switch, a power amplifier, a low noise amplifier (low noise amplifier, LNA), and the like. The mobile communication module 150 may receive an electromagnetic wave by using the antenna 1, perform processing such as filtering and amplification on the received electromagnetic wave, and send a processed electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules of the mobile communication module 150 may be disposed in the processor 110. In some embodiments, at least some functional modules of the mobile communication module 150 may be disposed in a same component as at least some modules of the processor 110.

The modem processor may include a modulator and a demodulator. The modulator is configured to adjust a to-be-sent low-frequency baseband signal to a medium/high-frequency signal. The demodulator is configured to demodulate a received electromagnetic wave signal into a low-frequency baseband signal. Then, the demodulator transmits the low-frequency baseband signal obtained through demodulation to the baseband processor for processing. After being processed by the baseband processor, the low-frequency baseband signal is transmitted to the application processor. The application processor outputs a sound signal by using an audio device (which is not limited to the speaker 170A, the receiver 170B, or the like), or displays an image or a video by using the display 194. In some embodiments, the modem processor may be an independent device. In some other embodiments, the modem processor may be independent of the processor 110 and disposed in a same device as the mobile communication module 150 or another functional module.

The wireless communication module 160 may provide a solution for wireless communication that is applied to the electronic device 200 and that includes a wireless local area network (wireless local area networks, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and the like. The wireless communication module 160 may be one or more components that integrate at least one communication processing module. The wireless communication module 160 receives an electromagnetic wave through the antenna 2, performs demodulation and filtering on an electromagnetic wave signal, and sends a processed signal to the processor 110. The wireless communication module 160 may further receive a to-be-sent signal from the processor 110, perform frequency modulation and amplification on the to-be-sent signal, and convert the to-be-sent signal into an electromagnetic wave for radiation through the antenna 2.

In some embodiments, in the electronic device 200, the antenna 1 is coupled to the mobile communication module 150, and the antenna 2 is coupled to the wireless communication module 160, so that the electronic device 200 can communicate with a network and another device according to a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, GNSS, WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation systems, SBAS).

The electronic device 200 implements a display function by using the GPU, the display 194, the application processor, and the like. The GPU is a microprocessor for image processing, and is connected to the display 194 and the application processor. The GPU is configured to perform mathematical and geometric calculation and render graphics. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flex light-emitting diode, FLED), a mini-LED, a micro-LED, a micro-OLED, a quantum dot light-emitting diode (quantum dot light-emitting diodes, QLED), or the like. In some embodiments, the electronic device 200 may include one or N displays 194, where N is a positive integer greater than 1.

The electronic device 200 may implement a photographing function by using the ISP, the camera 193, the video codec, the GPU, the display 194, the application processor, and the like.

The ISP is configured to process data fed back by the camera 193. For example, during photographing, a shutter is pressed, light is transmitted to a photosensitive element of the camera through a lens, an optical signal is converted into an electrical signal, and the photosensitive element of the camera transmits the electrical signal to the ISP for processing, to convert the electrical signal into a visible image. The ISP may further perform algorithm optimization on noise and luminance of the image. The ISP may further optimize parameters such as exposure and a color temperature of a photographing scenario. In some embodiments, the ISP may be disposed in the camera 193.

The camera 193 is configured to capture a still image or a video. An optical image of an object is generated by using a lens and projected onto a photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for conversion into a digital image signal. The ISP outputs the digital image signal to the DSP for processing. The DSP converts the digital image signal into a standard image signal in a format such as RGB or YUV. In some embodiments, the electronic device 200 may include one or N cameras 193, where N is a positive integer greater than 1.

The digital signal processor is configured to process a digital signal. In addition to processing a digital image signal, the digital signal processor may further process another digital signal. For example, when the electronic device 200 selects a frequency, the digital signal processor is configured to perform Fourier transform or the like on frequency energy.

The video codec is configured to compress or decompress a digital video. The electronic device 200 may support one or more types of video codecs. In this way, the electronic device 200 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor that processes input information rapidly by referring to a structure of a biological neural network, for example, by referring to a transmission mode between human brain neurons, and can further perform self-learning continuously. The NPU may be used to implement applications such as intelligent cognition of the electronic device 200, for example, image recognition, facial recognition, voice recognition, and text understanding.

The internal memory 121 may include one or more random access memories (random access memory, RAM) and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, where for example, a 5th generation DDR SDRAM is usually referred to as DDR5 SDRAM), and the like. The non-volatile memory may include a magnetic disk storage device and a flash memory (flash memory).

According to operation principles, the flash memory may include NOR FLASH, NAND FLASH, 3D NAND FLASH, and the like. According to quantities of potential levels of storage units, the flash memory may include a single-level cell (single-level cell, SLC), a multi-level cell (multi-level cell, MLC), a triple-level cell (triple-level cell, TLC), a quad-level cell (quad-level cell, QLC), and the like. According to storage specifications, the flash memory may include a universal flash storage (English: universal flash storage, UFS), an embedded multimedia card (embedded multimedia Card, eMMC), and the like.

The random access memory may be directly read and written by the processor 110, may be configured to store an executable program (for example, a machine instruction) of an operating system or another running program, and may be further configured to store data of a user and an application.

The non-volatile memory may also store an executable program, data of a user and an application, and the like, and may be loaded into the random access memory in advance, for direct reading and writing by the processor 110.

The external memory interface 120 may be configured to connect to an external non-volatile memory, to extend a storage capability of the electronic device 200. The external non-volatile memory communicates with the processor 110 by using the external memory interface 120, to implement a data storage function. For example, files such as music or videos are stored in the external non-volatile memory.

The electronic device 200 may implement an audio function, such as music playing and recording, by using the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to encode and decode audio signals. In some embodiments, the audio module 170 may be disposed in the processor 110, or some functional modules of the audio module 170 may be disposed in the processor 110.

The speaker 170A, also referred to as a "loudspeaker", is configured to convert an audio electrical signal into a sound signal. The electronic device 200 may be used to listen to music or answer a call in a hands-free mode over the speaker 170A.

The receiver 170B, also referred to as an "earpiece", is configured to convert an audio electrical signal into a sound signal. When a call is answered or a voice message is listened to by using the electronic device 200, the receiver 170B may be put close to a human ear to listen to a voice.

The microphone 170C, also referred to as a "mike" or a "mic", is configured to convert a sound signal into an electrical signal. When making a call or sending a voice message, the user may make a sound near the microphone 170C through the mouth, to enter a sound signal to the microphone 170C. At least one microphone 170C may be disposed on the electronic device 200. In some other embodiments, two microphones 170C may be disposed on the electronic device 200. In addition to sound signal collection, a noise reduction function may be further implemented. In some other embodiments, three, four, or more microphones 170C may be alternatively disposed on the electronic device 200, to collect a sound signal, implement noise reduction, recognize a sound source, implement a directional recording function, and the like.

The headset jack 170D is configured to connect to a wired headset. The headset jack 170D may be the USB interface 130, or may be a 3.5 mm open mobile terminal platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and may convert the pressure signal into an electrical signal. In some embodiments, the pressure sensor 180A may be disposed on the display 194. There are many types of pressure sensors 180A, such as a resistive pressure sensor, an inductive pressure sensor, and a capacitive pressure sensor.

The gyro sensor 180B may be configured to determine a motion gesture of the electronic device 200. In some embodiments, angular velocities of the electronic device 200 around the three axes (that is, the x-axis, the y-axis, and the z-axis) may be determined by using the gyro sensor 180B.

The barometric pressure sensor 180C is configured to measure barometric pressure.

The magnetic sensor 180D includes a Hall sensor.

The acceleration sensor 180E may detect values of acceleration of the electronic device 200 in all directions (usually on three axes). When the electronic device 200 is static, the acceleration sensor can detect magnitude and a direction of gravity. The acceleration sensor may be further configured to recognize a posture of the terminal device.

The distance sensor 180F is configured to measure a distance. The electronic device 200 may measure a distance through infrared or laser.

The optical proximity sensor 180G may include, for example, a light-emitting diode (LED) and a light detector such as a photodiode.

The ambient light sensor 180L is configured to sense ambient light brightness. The electronic device 200 may adaptively adjust brightness of the display 194 based on the sensed ambient light brightness.

The fingerprint sensor 180H is configured to collect a fingerprint.

The temperature sensor 180J is configured to detect a temperature. In some embodiments, the electronic device 200 executes a temperature processing policy based on the temperature detected by the temperature sensor 180J.

The touch sensor 180K is also referred to as a "touch device". The touch sensor 180K may be disposed on the display 194. The touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of a touch event. Visual output related to the touch operation may be provided by using the display 194. In some other embodiments, the touch sensor 180K may alternatively be disposed on a surface of the electronic device 200 at a position different from that of the display 194.

The bone conduction sensor 180M may obtain a vibration signal. In some embodiments, the bone conduction sensor 180M may obtain a vibration signal of a vibration bone of a human vocal cord part.

The button 190 includes a power button, a volume button, and the like. The button 190 may be a mechanical button, or may be a touch button.

The motor 191 may generate a vibration prompt.

The indicator 192 may be an indicator light, and may indicate a charging status, a power change, a message, a missed call, or the like.

The SIM card interface 195 is configured to connect to a SIM card.

The following describes an example of an application scenario and a related implementation of the menstrual period management method provided in embodiments of this application.

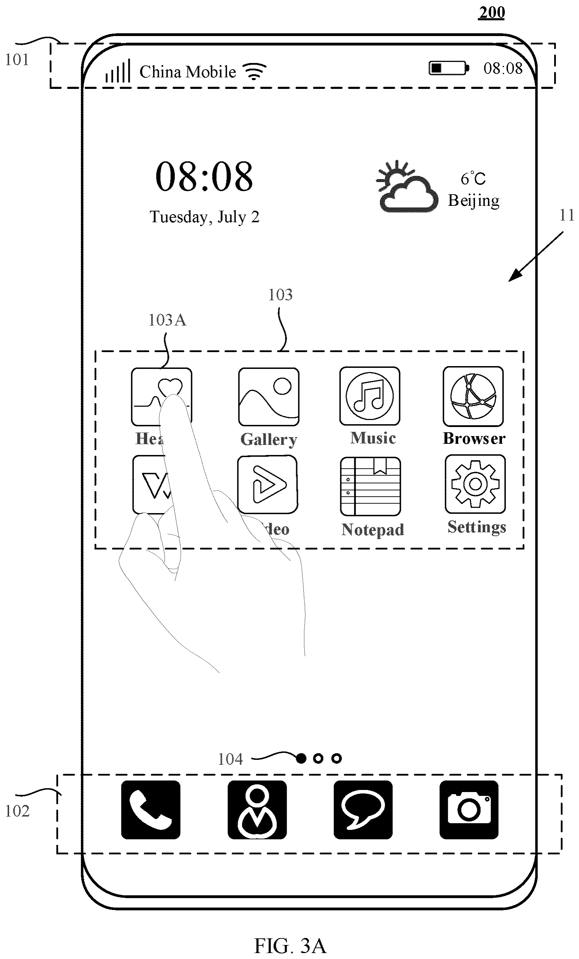

In some application scenarios, an app used for menstrual period management is installed on the electronic device 200 and/or the smart band 100. For example, the app is a Health app. For example, FIG. 3A to FIG. 3H show related user interfaces of the Health app on the electronic device 200.

For example, FIG. 3A shows a home screen 11 that is on the electronic device 200 and that is used to display an installed application (application, APP). The home screen 11 may include a status bar 101, a tray 102 including a frequently used application icon, and other application icons 103. The tray 102 including a frequently used application icon may display a Phone icon, a Contacts icon, a Messages icon, and a Camera icon. The other application icons 103 may display a Health icon 103A, a Gallery icon, a Music icon, a Settings icon, and the like. The home screen 11 may further include a page indicator 104. The other application icons may be distributed on a plurality of pages. The page indicator 104 may indicate a page on which an application currently viewed by the user is located. The user may slide leftward or rightward in a region of the other application icons to view application icons on another page.

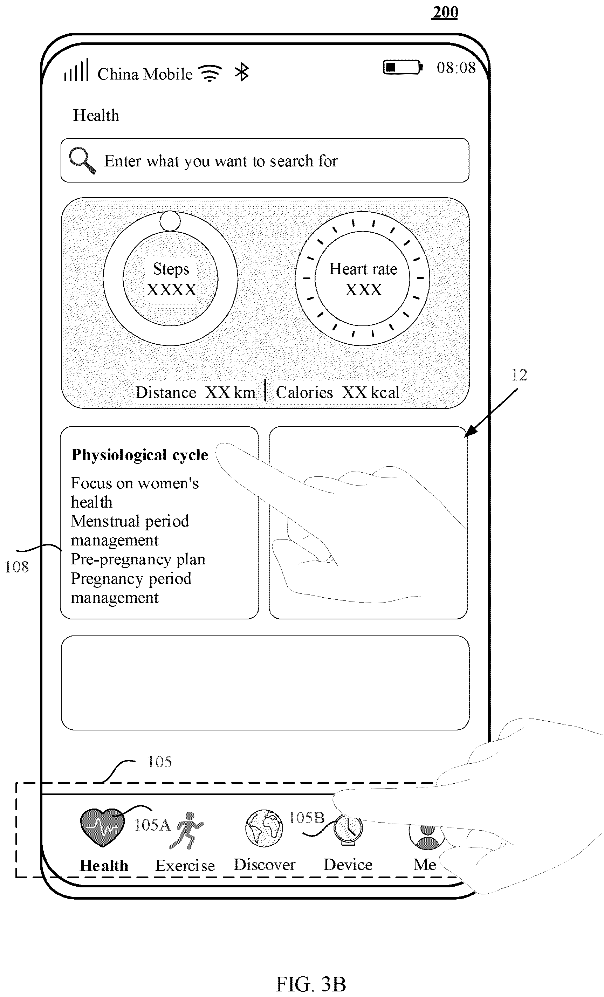

As shown in FIG. 3A and FIG. 3B, after detecting that a user taps the Health icon 103A of the Health app, the electronic device 200 displays a user interface 12 of the Health app. The user interface 12 includes a menu bar 105, and the menu bar 105 includes a plurality of menu options, such as a health option 105A, a device option 105B, and a user option. The user interface 12 is a user interface corresponding to the health option 105A.

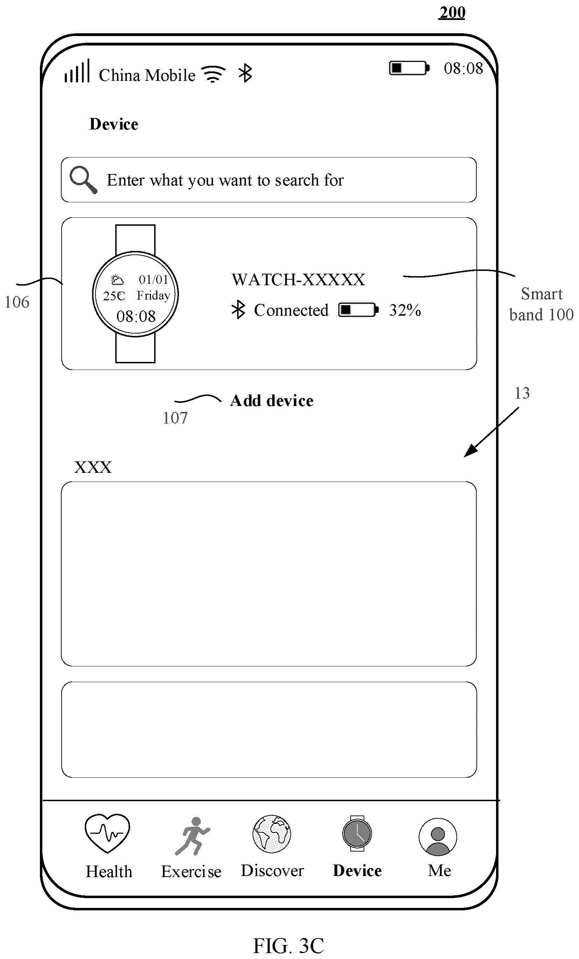

As shown in FIG. 3B and FIG. 3C, after detecting that the user taps the device option 105B, the electronic device 200 may display a user interface 13 corresponding to the device option 105B, and the user interface 13 is used to display a paired and bound device. The electronic device 200 has been paired with and bound to the smart band 100, and the user interface 13 displays an information bar 106 of the smart band 100. The information bar 106 may include a device model of the smart band 100, and may further include information such as a remaining battery level of the smart band 100 and a communication connection manner (for example, a Bluetooth connection) to the electronic device 200. The user interface 13 further includes a device adding control 107, and the device adding control 107 is configured to trigger pairing and binding between the electronic device 200 and another device (for example, another detection apparatus).

In some embodiments, the electronic device 200 may obtain physiological data of a plurality of physiological features collected by the smart band 100, to obtain daily physiological features of the user based on the physiological data. The plurality of physiological features may include some or all of the following: one or more indicators of a body temperature, a heart rate, and a heart rate variability. A daily menstrual period trend value of the user may be determined based on data of a plurality of physiological features of the user obtained recently (for example, in 30 days before a current date). The menstrual period trend value indicates a degree to which the current date is close to a peak of the menstrual period. Based on a menstrual period trend value obtained on a current day, it may be predicted whether today is a first menstrual day or a last menstrual day. Based on recent menstrual period trend values of the user and menstrual period trend values of historical physiological cycles, first days and last days of one or more menstrual periods in the future may be predicted, so that an ovulatory period and a fertile period can be predicted. According to individual differences, dates corresponding to menstrual peaks in menstrual periods of different users (that is, dates with largest menstrual period trend values) may be different. For example, a length of a menstrual period is 6, and the peak of the menstrual period is on the third day of the menstrual period. Generally, if the current date is closer to the peak of the menstrual period, the menstrual period trend value of the current date is larger. Due to impact of various factors, there may be a fluctuation.

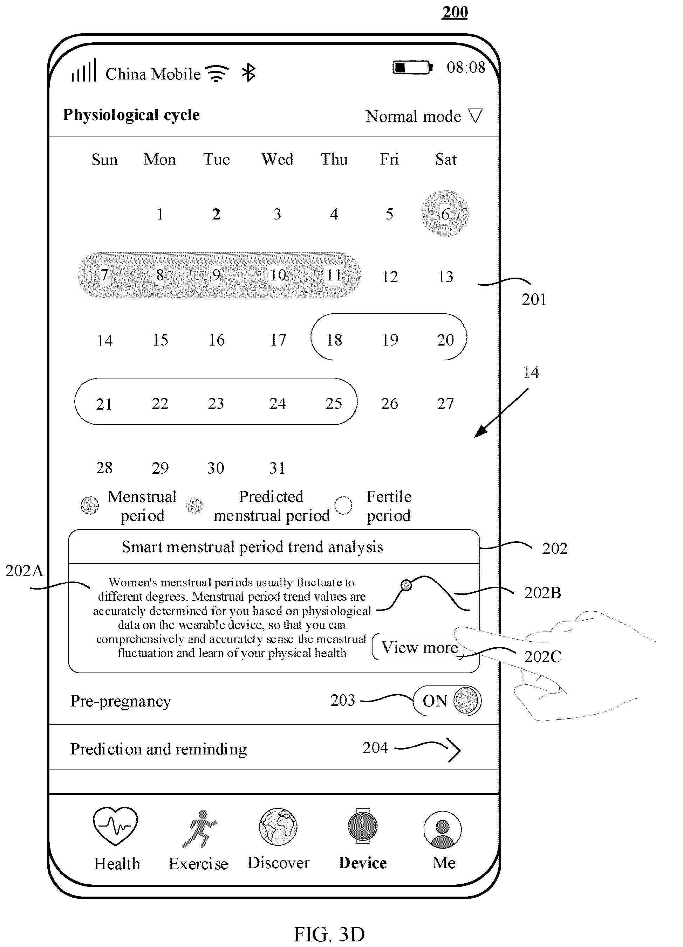

As shown in FIG. 3B and FIG. 3D, the user interface 12 includes a physiological cycle card 108, and the physiological cycle card 108 is used to implement some or all of the following: menstrual period management, pregnancy preparation planning, pregnancy period management, and the like. After detecting that the user taps the card 108, the electronic device 200 may display a user interface 14 of the physiological cycle. The user interface 14 includes a calendar 201, and the calendar 201 indicates a date range corresponding to a menstrual period (for example, a menstrual period 1) in a current month. If the current date does not reach a first menstrual day, the menstrual period indicated by the calendar 201 is a menstrual period predicted based on a menstrual period trend value of a historical physiological cycle. If the current date reaches or exceeds a first menstrual day, the first menstrual day indicated by the calendar 201 is determined based on a recently measured menstrual period trend value or is determined through manual correction of the user. If the current date reaches or exceeds a last menstrual day, the last menstrual day indicated by the calendar 201 is determined based on a recently measured menstrual period trend value or is determined through manual correction of the user.

In some embodiments, the calendar 201 further indicates some or all of the following special periods: a follicular period, an ovulatory period, a luteal period, a fertile period, a safe period, and a pregnancy preparation period. The special periods may be determined based on predicted and calibrated menstrual periods. In some embodiments, the user interface 12 includes a switch control 203 for pregnancy preparation, and the switch control 203 includes two states: an on state and an off state. The calendar 201 indicates the foregoing special periods only when the switch control 203 is in the on state.

In some embodiments, the user interface 12 includes a card 202 for intelligently analyzing a menstrual period trend. The card 202 may include some or all of introduction information 202A of a function of intelligently analyzing a menstrual period trend, a diagram 202B of a menstrual period trend curve, and a details control 202C. For example, the foregoing introduction information includes "Women's menstrual periods usually fluctuate to different degrees. Menstrual period trend values are accurately determined for you based on physiological data on the wearable device, so that you can comprehensively and accurately sense the menstrual fluctuation and learn of your physical health". The diagram 202B of the menstrual period trend curve indicates a menstrual period trend curve within a physiological cycle, and a location of the menstrual period trend value of the current date in the menstrual period trend curve.

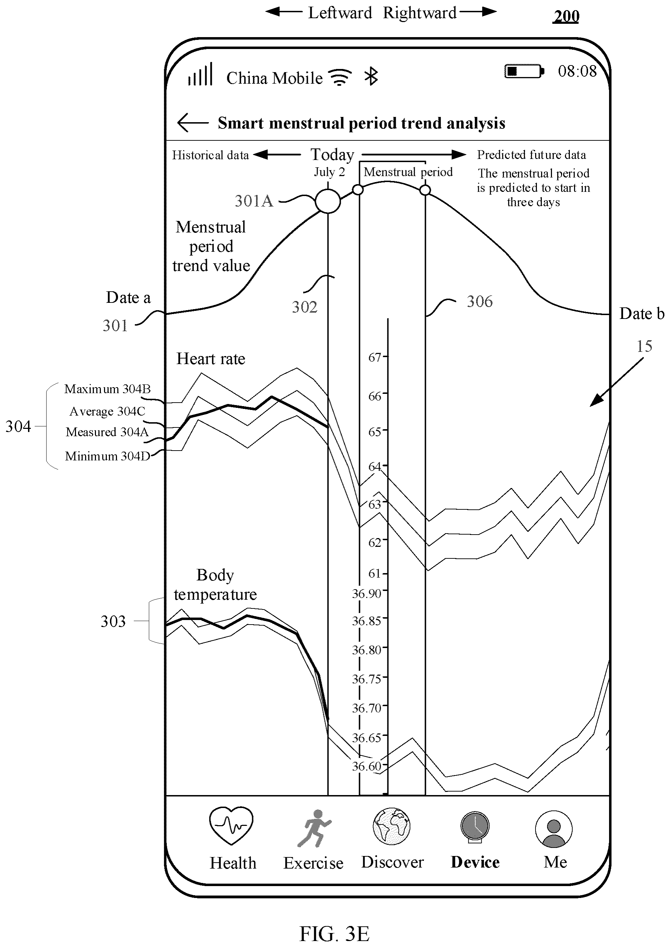

As shown in FIG. 3D and FIG. 3E, after detecting that the user taps the details control 202C, the electronic device 200 may display a user interface 15 for intelligently analyzing a menstrual period trend. The user interface 15 includes a menstrual period trend curve 301 from a date a to a date b. A horizontal coordinate of the menstrual period trend curve 301 may be the date. The date a to the date b include the current date, that is, a date c (for example, July 2). A vertical coordinate may be the menstrual period trend value. In some embodiments, the user interface 15 further includes a vertical axis 302, the vertical axis 302 is a vertical axis corresponding to the current date, and a vertical coordinate of an intersection point of the vertical axis 302 and the menstrual period trend curve 301 is a detected menstrual period trend value corresponding to the current date. In some embodiments, the date a to the date b include a physiological cycle, for example, 28 days.

With reference to FIG. 3E, menstrual period trend values of the current date and previous dates on the menstrual period trend curve 301 are actual values detected based on physiological features of the user, and menstrual period trend values after the current date are predicted values determined based on menstrual period trend values in historical physiological cycles.

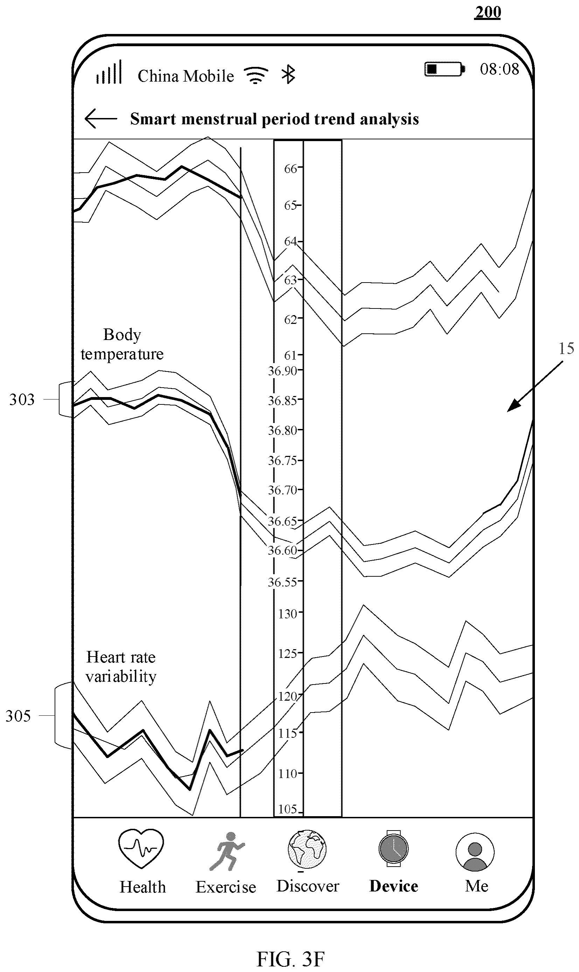

In some embodiments, for some or all of one or more physiological features, the user interface 15 further includes a measured physiological feature curve corresponding to each physiological feature within the date a to the date c (that is, the current date), and a historical physiological feature curve corresponding to each physiological feature within the date a to the date b, for example, a body temperature curve 303, a heart rate curve 304, and a heart rate variability curve 305 corresponding to an indicator 1 of a heart rate variability shown in FIG. 3E and FIG. 3F. As shown in FIG. 3E and FIG. 3F, when not all curves can be displayed due to a limited screen size, the user interface 15 may be slid up to view more curves in the user interface 15. A horizontal coordinate of each physiological feature curve is in one-to-one correspondence with a horizontal coordinate of a menstrual period trend value curve, and a vertical coordinate is a physiological feature value corresponding to a date.

A historical physiological feature curve corresponding to a physiological feature includes one or more of the following physiological feature curves: a measured physiological feature curve, a historical minimum physiological feature curve, a historical average physiological feature curve, and a historical maximum physiological feature curve. The measured physiological feature curve indicates physiological feature values measured on the current date and a previous date; the historical minimum physiological feature curve indicates a minimum physiological feature value on every day in one historical physiological cycle obtained through comprehensive statistics collection; the historical average physiological feature curve indicates an average physiological feature value on every day in one historical physiological cycle obtained through comprehensive statistics collection; and the historical maximum physiological feature curve indicates a maximum physiological feature value on every day in one historical physiological cycle obtained through comprehensive statistics collection. The heart rate curve 304 corresponding to the heart rate is used as an example. The heart rate curve 304 includes a measured heart rate curve 304A from the date a to the date c, and further includes three historical physiological feature curves: a historical maximum heart rate curve 304B, a historical average heart rate curve 304C, and a historical minimum heart rate curve 304D. Each historical physiological feature curve is determined based on physiological feature values in a historical physiological cycle. A historical physiological feature curve in a subsequent embodiment is the foregoing historical average heart rate curve.

In some embodiments, in response to an operation of sliding a curve display area in the user interface 15 rightward by the user, the electronic device 200 may display a measured menstrual period trend curve and a measured physiological feature curve that are corresponding to a date before the date a, so that the user views a menstrual period trend value and a physiological feature of a more distant past date. In response to an operation of sliding the curve display area in the user interface 15 leftward, the electronic device 200 may display a predicted menstrual period trend curve and a predicted physiological feature curve that are corresponding to a date after the date b, so that the user views a predicted menstrual period trend value and a predicted physiological feature of a more distant future date. The curve display area includes a display area of a menstrual period trend curve and each physiological feature curve. It may be understood that the foregoing leftward and rightward are relative concepts in the examples provided in embodiments of this application, and should not be construed as a limitation on embodiments of this application.

In some embodiments, the user interface 15 further includes a menstrual period indicator 306, and the menstrual period indicator indicates a date range of a menstrual period 1 from the date a to the date b. As shown in FIG. 3E, the menstrual period indicator 306 may be presented as a box, and the box includes a menstrual period trend curve and each physiological feature curve in the menstrual period 1. A date range of the menstrual period 1 indicated by the calendar 201 in the user interface 14 is the same as a date range indicated by the menstrual period indicator 306.

In some embodiments, as shown in FIG. 3E, an indicator 301A is displayed on the menstrual period trend curve 301, and the indicator 301A indicates a point corresponding to the current date on the menstrual period trend curve 301, that is, an intersection point of a vertical axis 302 of the current date and the menstrual period trend curve 301. Optionally, prompt information is displayed near the indicator 301A, to indicate that symptom details may be viewed by using the indicator 301A.

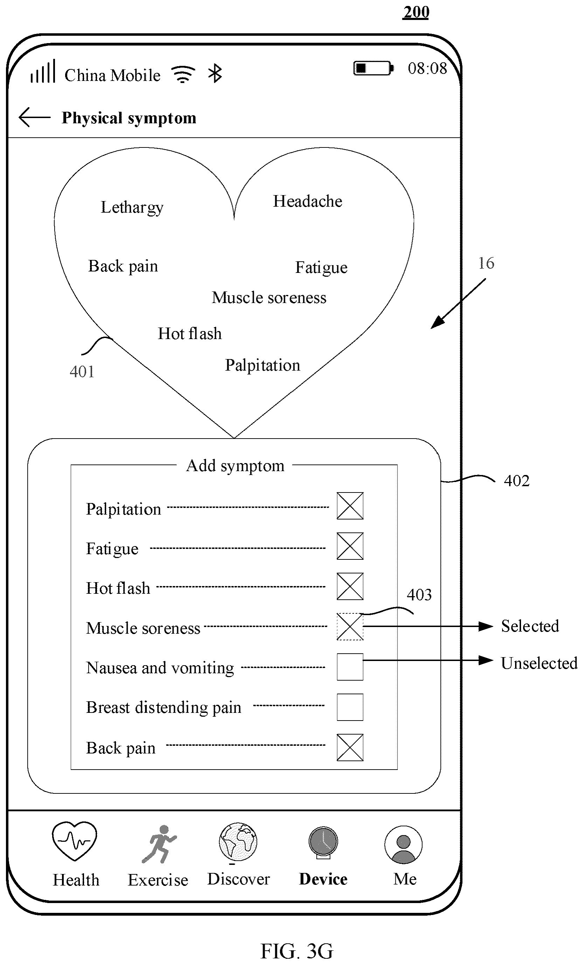

As shown in FIG. 3E and FIG. 3G, after detecting that the user taps the indicator 301A, the electronic device 200 displays a user interface 16. A display area 401 of the user interface 16 is used to display a physical symptom that may exist on the current date, for example, heart palpitation, fatigue, hot flush, and muscle soreness. The user interface 16 may further include a symptom editing box 402, where the symptom editing box 402 is used to add and/or delete a symptom displayed in the display area 401. As shown in FIG. 3G, the symptom editing box 402 displays selection controls respectively corresponding to all symptoms, for example, a selection control 403 corresponding to muscle soreness. The selection control 403 has two states: selected and unselected. The user may tap the selection control 403 to switch the selection control 403 between the two states. When the selection control 403 is switched from the selected state to the unselected state, a corresponding symptom is deleted from the display area 401; or when the selection control 403 is switched from the unselected state to the selected state, a corresponding symptom is added to the display area 401. A manner of adding and/or deleting a symptom by the user is not specifically limited in embodiments of this application.

Physical symptoms of the user may be different in different periods (for example, a menstrual period and an ovulatory period) in a physiological cycle and on different days (for example, a fifth day of the menstrual period) in a period. In an implementation, a physical symptom corresponding to each day (for example, a day of the date c) in a physiological cycle (for example, 28) is preset in the Health app. For example, a period 1 (for example, a menstrual period) to which the date c belongs and a location (for example, a fifth day) of the date c in the period 1 are considered for the preset physical symptom corresponding to the date c. The electronic device 200 may display the physical symptom preset in the app in the display area 401. On this basis, a physical symptom corresponding to a day of the date c in a physiological cycle may be updated based on the symptom added/deleted by the user. In an implementation, the user may record a daily physical symptom by using the Health app. A physical symptom corresponding to each day in a physiological cycle that is preset in the Health app is determined based on the physical symptom recorded by the user. If the user does not record a physical status on a day, a preset symptom on the day may be null.

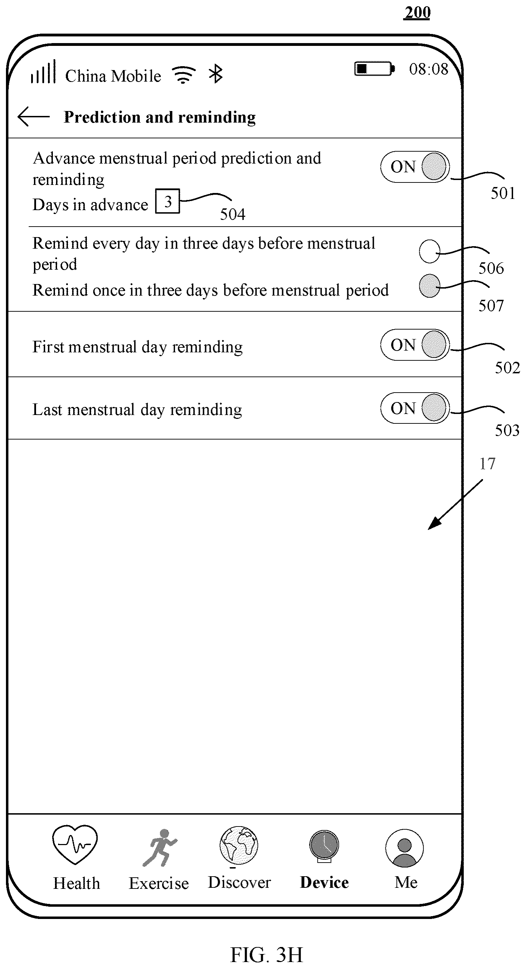

In some embodiments, as shown in FIG. 3D, the user interface 14 further includes a setting bar 204 for prediction and reminding, and the setting bar 204 for prediction and reminding is used to set a reminder for a physiological cycle. As shown in FIG. 3D and FIG. 3H, after detecting that the user taps the setting bar 204, the electronic device 200 displays a setting interface 17. The setting interface 17 includes switch controls of one or more reminders, for example, a switch control 501 for advance prediction and reminding for a menstrual period, a switch control 502 for a reminder for a first menstrual day, and a switch control 503 for a reminder for a last menstrual day. The switch control (for example, the switch control 501) has two states: on and off. The user taps the switch control, so that the switch control can be switched between the two states. When a switch control corresponding to a reminder is switched from the off state to the on state, the reminder is enabled. When a switch control corresponding to a reminder is switched from the on state to the off state, the reminder is disabled.

In some embodiments, as shown in FIG. 3H, when the switch control 501 for advance prediction and reminding for a menstrual period is in the on state, the user interface 16 further includes a setting control 504, where the setting control 504 is configured to receive a quantity X of days for advance reminding that is set by the user. For example, as shown in FIG. 3H, the quantity of days set through the setting control 504 is 3. If the electronic device 200 predicts that the menstrual period arrives after/within three days, the electronic device 200 outputs advance prompt information, to remind the user in advance that the menstrual period arrives after/within three days. In some embodiments, as shown in FIG. 3H, when the switch control 501 for advance prediction and reminding for a menstrual period is in the on state, the user interface 16 further includes a timing option for advance prediction and reminding, for example, a timing option 506 and a timing option 507. The timing option 506 indicates that a timing for advance prediction and reminding is the X^{th} day (for example, the third day) before the menstrual period. The timing option 506 indicates that a timing for advance prediction and reminding for the menstrual period is each of X days (for example, the third day) before the menstrual period. That is, when it is detected that a quantity of days to the menstrual period is less than or equal to X, prompt information is output. Only one of the timing option 506 and the timing option 507 is in a selected state. The user may switch the currently selected timing option by tapping the timing option.

FIG. 3A to FIG. 3H show an example in which the user adds a physical symptom and sets a reminder for a physiological cycle. Operations of adding a physical symptom and setting a reminder are not specifically limited in embodiments of this application, and may also be implemented by using other operations.

In some embodiments, the prompt information (for example, advance prompt information) may be output by using the electronic device 200 and/or the smart band 100. In an implementation, when the electronic device 200 detects that a timing for advance prediction and reminding for the menstrual period arrives, the electronic device 200 sends a request message to the smart band 100. The request message indicates the smart band 100 to output advance prompt information. In an implementation, the smart band 100 outputs advance prompt information when detecting that a timing for advance prediction and reminding for the menstrual period arrives. In an implementation, Health apps on the electronic device 200 and the smart band 100 log in to a same account, and a server of the Health app performs menstrual period prediction based on a physiological feature of the user. When detecting that a timing for advance prediction and reminding for the menstrual period arrives, the server sends a request message to the electronic device 200 and/or the smart band 100. The request message indicates to output advance prompt information. In an implementation, Health apps on the electronic device 200 and the smart band 100 log in to a same account, and the electronic device 200 performs menstrual period prediction based on a physiological feature of the user. When detecting that a timing for advance prediction and reminding for the menstrual period arrives, the electronic device 200 sends a request message to the smart band 100 by using a server of the Health app. The request message indicates the smart band 100 to output advance prompt information. Similarly, for first-day prompt information and last-day prompt information in subsequent embodiments, refer to the related implementation of the foregoing advance prompt information.

In some embodiments, along with the advance prompt information, a confirmation control and a correction control are further displayed. The confirmation control indicates the user to confirm that the user has learned of the advance prompt information, and the correction control indicates that the menstrual period has started and is used to calibrate a date of a first menstrual day. It may be understood that, due to impact of various factors, the menstrual period fluctuates, and the following case may exist: "When the user is reminded X days in advance that the menstrual period is to arrive, the menstrual period of the user has arrived". In this case, the user may manually feed back the first day of the current menstrual period by using the correction control. The user can be reminded, by using the advance prompt information for the menstrual period, in time that the menstrual period is to arrive, so that the user makes psychological preparation, material preparation, and trip arrangement in advance.

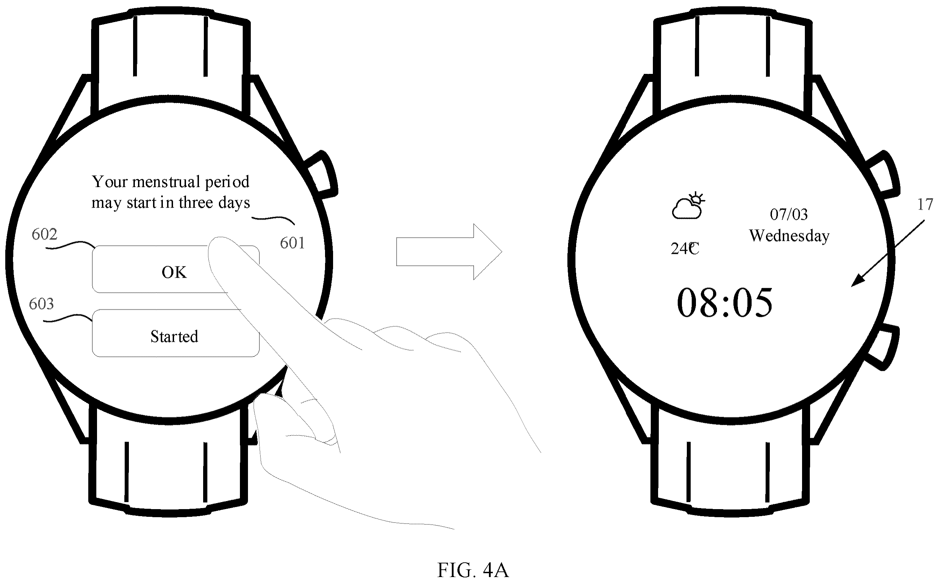

For example, FIG. 4A to FIG. 4C show related user interfaces of advance prediction and reminding of the smart band 100.

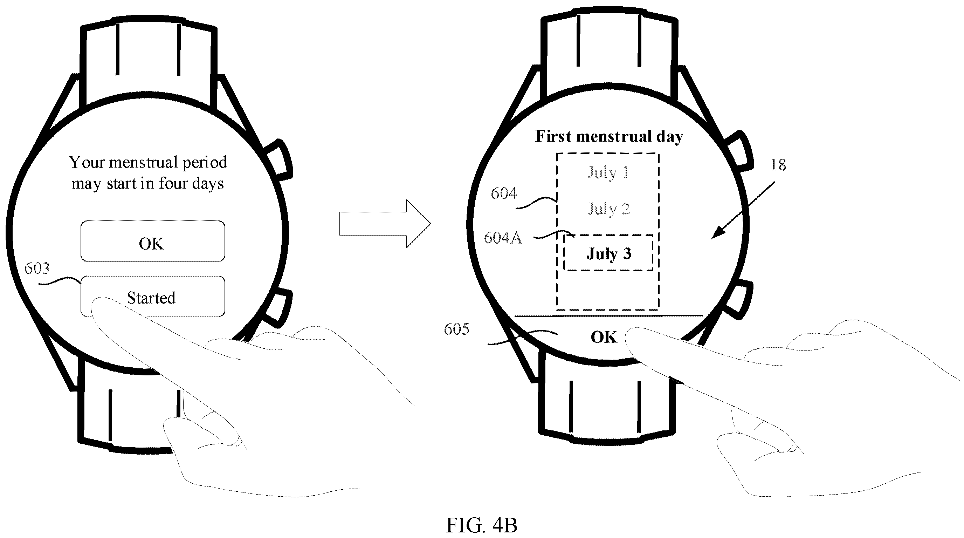

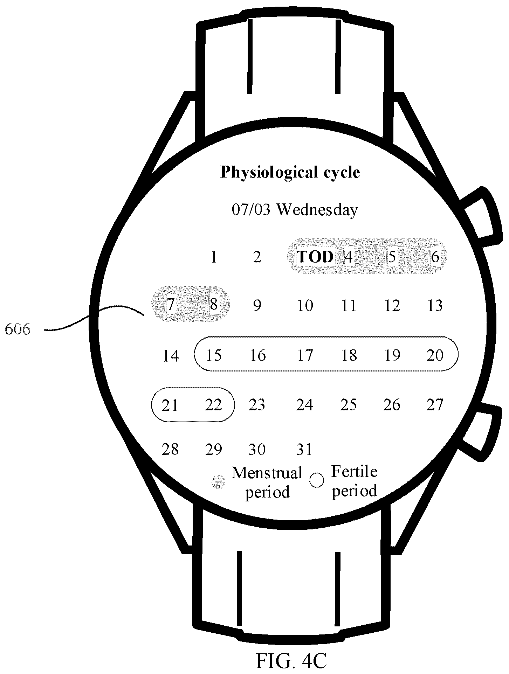

As shown in FIG. 4A, on July 3, the smart band 100 displays advance prompt information 601, a confirmation control 602, and a correction control 603. As shown in FIG. 4A, presented content of the confirmation control 602 may include "OK". After detecting that the user taps the confirmation control 602, the smart band 100 displays a home screen 17, returns to a previous interface (that is, an interface displayed before the advance prompt information 601 is displayed), or displays a calendar of a physiological cycle. As shown in FIG. 4B, presented content of the correction control 603 may include "The menstrual period has started". After detecting that the user taps the correction control 603, the smart band 100 displays a user interface 18. The user interface 18 includes a date correction control 604 and a confirmation control 605. The date correction control 604 is configured to select a first menstrual day. In an implementation, an area 604A in the date correction control 604 is used to display a selected date. The user may slide up or down in a display area of the date correction control 604 to view more dates, and move a date of an actual first menstrual day to the area 604A. As shown in FIG. 4C, after detecting an operation (for example, a tap operation) performed by the user on the confirmation control 605, the smart band 100 calibrates a date currently displayed in the area 604A(that is, July 3) as the first menstrual day, and displays a calendar 606 of the physiological cycle. The calendar 606 of the physiological cycle indicates a first menstrual day corrected by the user.

In some embodiments, as shown in FIG. 3H, when the switch control 502 for a reminder for a first menstrual day is in an on state, if the first menstrual day is detected, first-day prompt information is output, to prompt the user that the first menstrual day has been detected and preliminary calibration is completed. Completing the preliminary calibration may include: recording the detected first menstrual day, and marking the date as the first menstrual day of the menstrual period in the calendar of the physiological cycle. The calibrated first menstrual day and a corresponding menstrual period trend value are used as historical data, to implement subsequent menstrual period prediction. In some embodiments, along with the first-day prompt information, a confirmation control and a correction control are further displayed. The confirmation control indicates the user to confirm that the user has learned of the first-day prompt information, and the correction control is used to manually correct a date of an automatically calibrated first menstrual day.

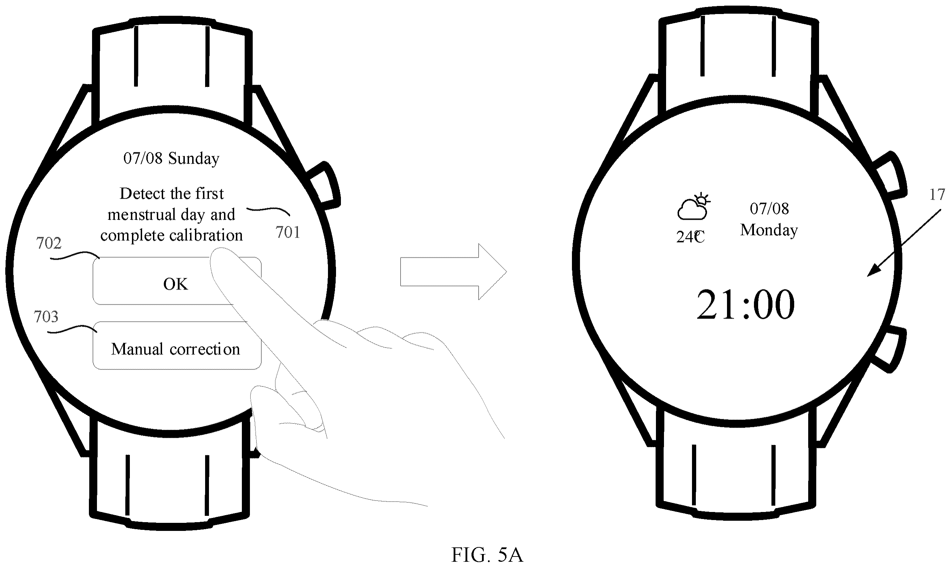

For example, FIG. 5A to FIG. 5E show related user interfaces of first menstrual day reminding of the smart band 100.

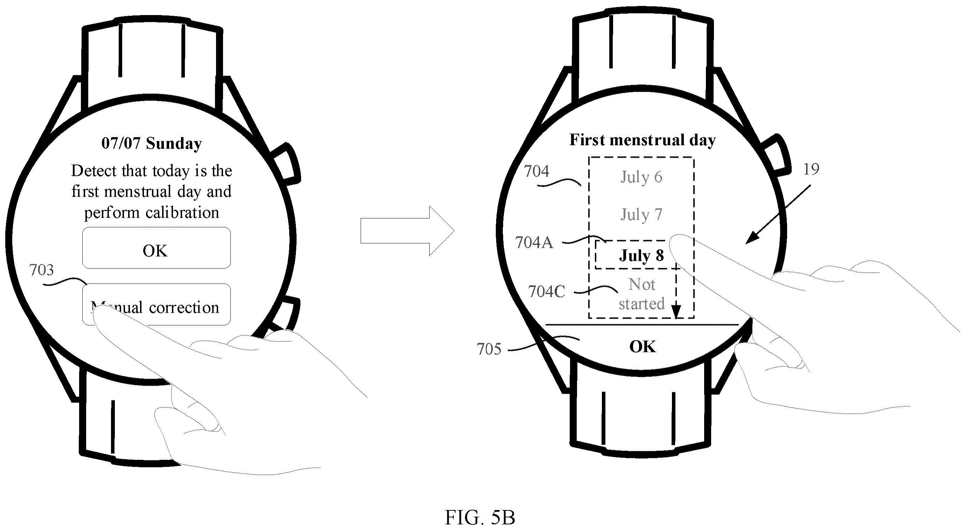

As shown in FIG. 5A, the smart band 100 displays first-day prompt information 701, a confirmation control 702, and a correction control 703. As shown in FIG. 5A, after detecting that the user taps the confirmation control 702, the smart band 100 displays a home screen 17, returns to a previous interface (that is, an interface displayed before the first-day prompt information 701 is displayed), or displays a calendar of a physiological cycle. As shown in FIG. 5B, after detecting that the user taps the correction control 703, the smart band 100 displays a user interface 19. The user interface 19 includes a date correction control 704 and a confirmation control 705. The date correction control 704 is configured to select a first menstrual day.

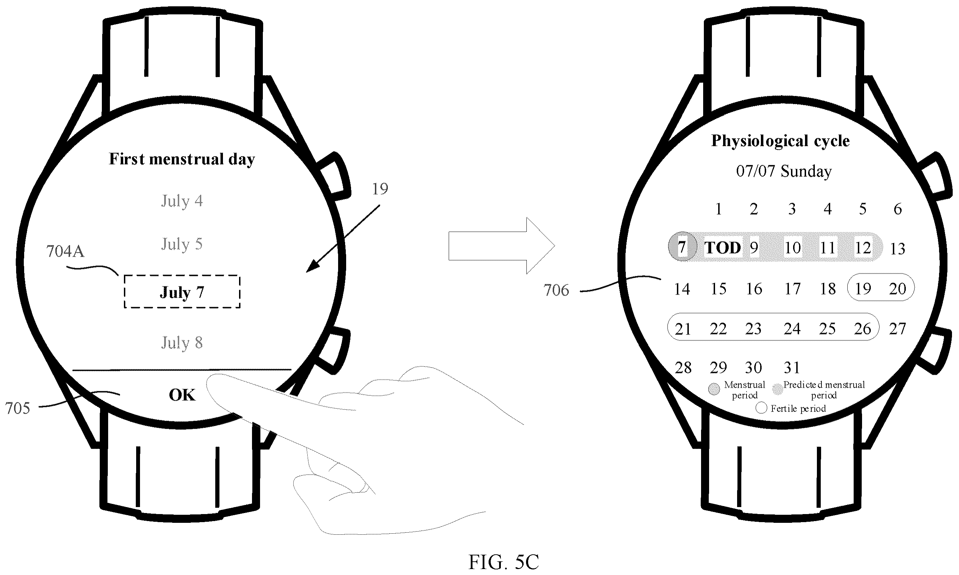

In some embodiments, the date correction control 704 includes date options (for example, July 7) of the current date and a previous date. An area 704A in the date correction control 704 is used to display a selected date. A finger of the user may slide up or down in the display area of the date correction control 704 to view more dates, and move a date of an actual first menstrual day to the area 704A. As shown in FIG. 5B and FIG. 5C, when a downward slide operation performed on the date correction control 704 is detected, a date option corresponding to July 7 is moved to the area 704A. After an operation (for example, a tap operation) performed on the confirmation control 705 is detected, the smart band 100 updates a calibrated first menstrual day (for example, July 8) of a latest menstrual period to the date (that is, July 7) currently displayed in the area 704A, and displays a calendar 706 of the physiological cycle. The calendar 706 of the physiological cycle indicates the first menstrual day corrected by the user.

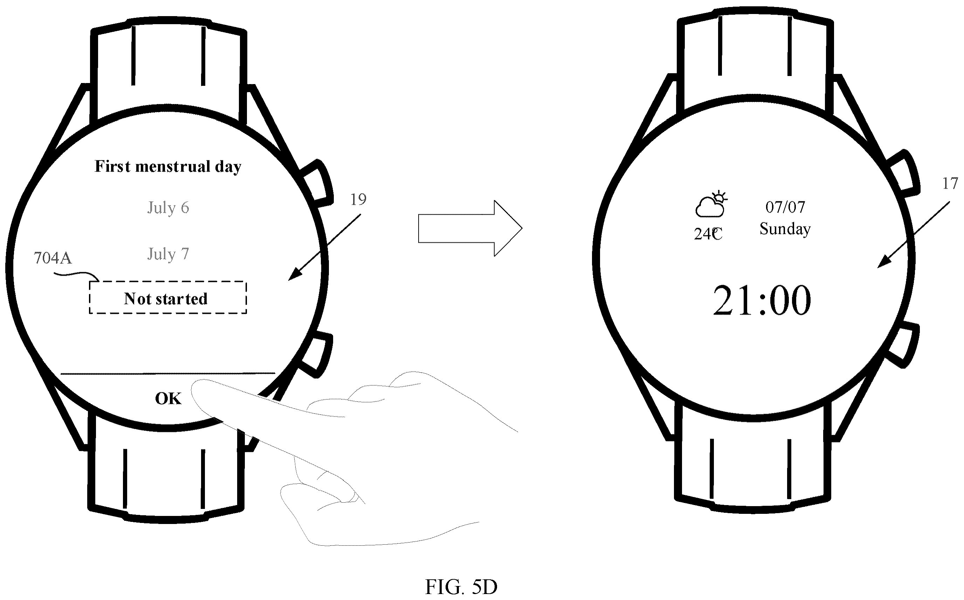

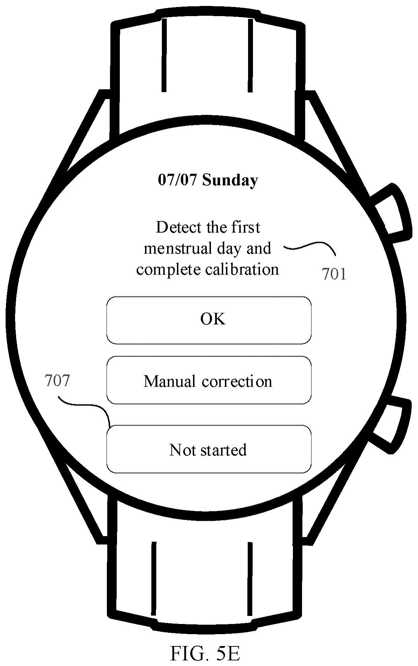

In some embodiments, with reference to FIG. 5B, the date correction control 704 may further include an option 704C used to indicate that the menstrual period does not start, and the calibrated first menstrual day of the latest menstrual period may be canceled by using the option 704C. With reference to FIG. 5B and FIG. 5D, when an upward slide operation performed on the date correction control 704 is detected, the option 704C is moved to the area 704A. After an operation (for example, a tap operation) performed on the confirmation control 705 is detected, the smart band 100 cancels the calibrated first menstrual day (for example, July 8) of the latest menstrual period. In some embodiments, the option 704C used to indicate that the menstrual period does not start does not need to be displayed in the date correction control 704. As shown in FIG. 5E, a correction control 707 used to indicate that the menstrual period does not start may be directly displayed along with the first-day prompt information 701. After detecting an operation (for example, a tap operation) performed by the user on the correction control 707, the smart band 100 directly cancels the calibrated first menstrual day of the latest menstrual period. It may be understood that, after the user feeds back that the menstrual period does not start, the first menstrual day of the current menstrual period continues to be detected, and a reminder is sent again after the first menstrual day is detected.

In some embodiments, as shown in FIG. 3H, when the switch control 503 for a reminder for a last menstrual day is in an on state, if the last menstrual day is detected, last-day prompt information is output, to prompt the user that the last menstrual day has been detected and preliminary calibration is completed. Completing the preliminary calibration may include: recording the detected last menstrual day, and marking the date as the last menstrual day of the menstrual period in the calendar of the physiological cycle. The calibrated last menstrual day and a corresponding menstrual period trend value are used as historical data, to implement subsequent menstrual period prediction. In some embodiments, along with the last-day prompt information, a confirmation control and a correction control are further displayed. The confirmation control indicates the user to confirm that the user has learned of the last-day prompt information, and the correction control is used to manually correct a date of an automatically calibrated last menstrual day.

In an implementation, if it is detected that the current date is the last menstrual day, the last-day prompt information is output on the current date. In another implementation, considering that the menstrual blood flow at the end of the menstrual period is discontinuous, to avoid that the user cannot confirm whether the menstrual period can completely end on the current date, the last-day prompt information is output only when it is detected that a date is the Y^{th} (for example, 1, 2, or 3) day after the last menstrual day. The last-day prompt information indicates a last day detected in 3.

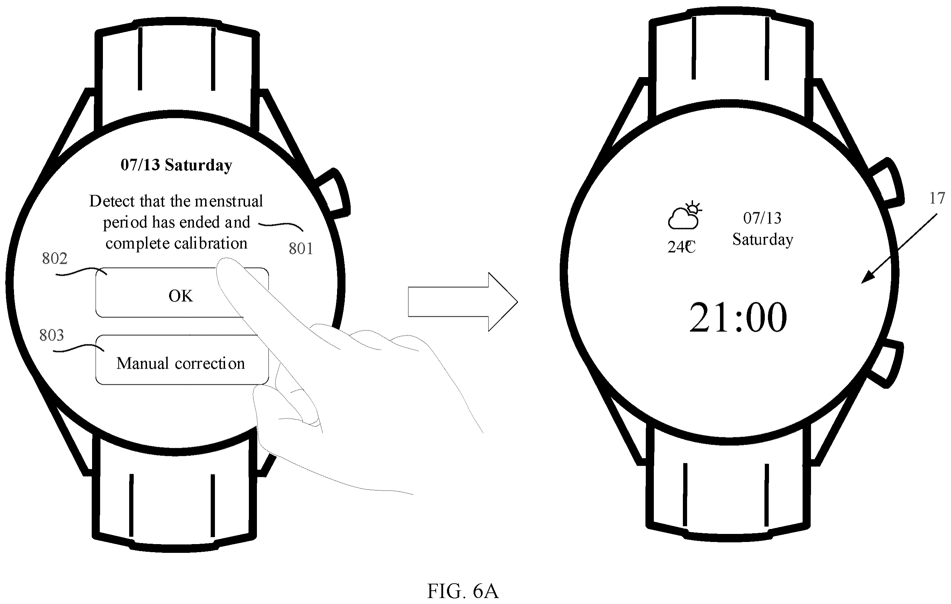

For example, FIG. 6A to FIG. 6E show related user interfaces of last menstrual day reminding of the smart band 100.

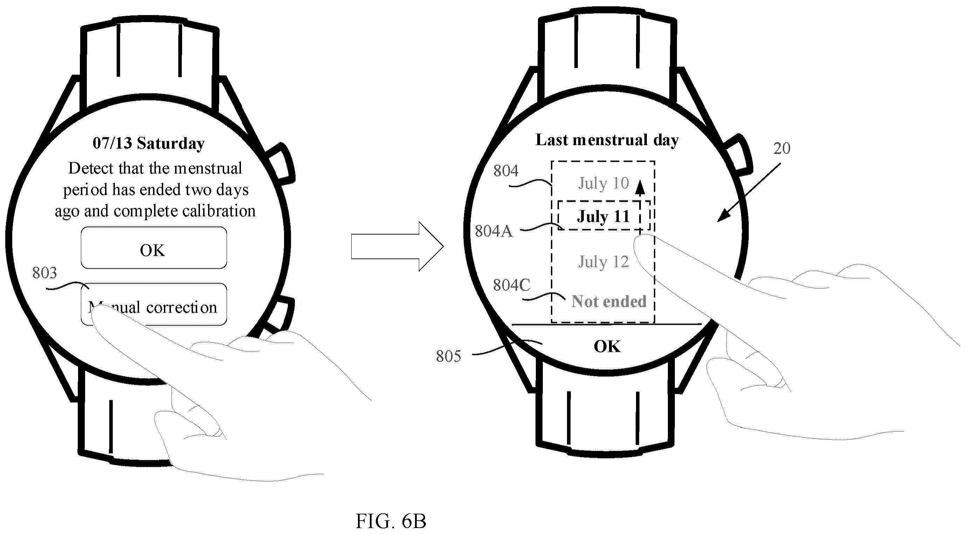

As shown in FIG. 6A, the smart band 100 displays last-day prompt information 801, a confirmation control 802, and a correction control 803. As shown in FIG. 6A, after detecting that the user taps the confirmation control 802, the smart band 100 displays a home screen 17, returns to a previous interface (that is, an interface displayed before the last-day prompt information 801 is displayed), or displays a calendar of a physiological cycle. As shown in FIG. 6B, after detecting that the user taps the correction control 803, the smart band 100 displays a user interface 20. The user interface 20 includes a date correction control 804 and a confirmation control 805. The date correction control 804 is configured to select a last menstrual day.

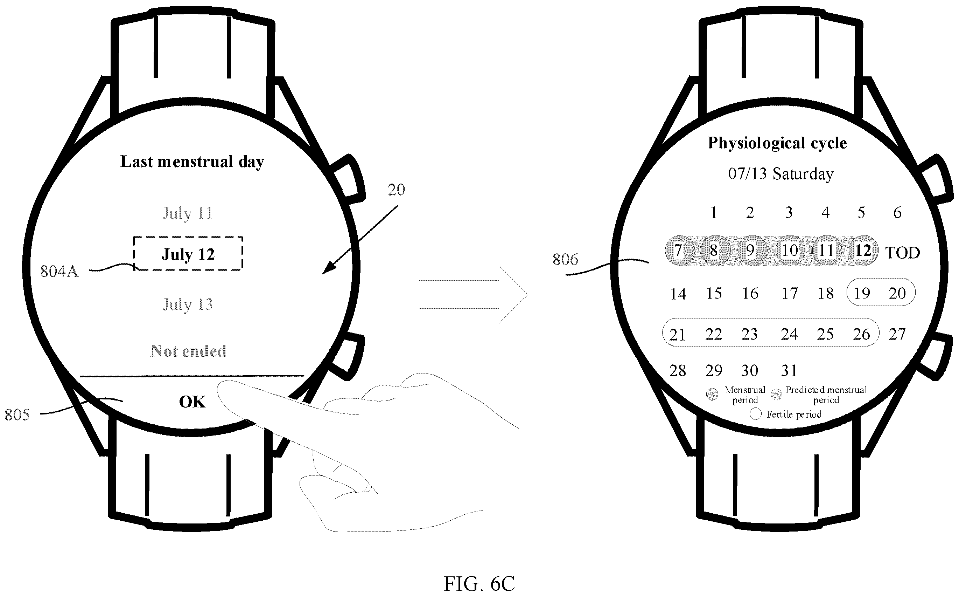

In some embodiments, the date correction control 804 includes date options of a current date (for example, July 13) and a previous date, and an area 804A in the date correction control 804 is used to display a selected date. A finger of the user may slide up or down in the display area of the date correction control 804 to view more dates, and move a date of an actual last menstrual day to the area 804A. As shown in FIG. 6B and FIG. 6C, when a downward slide operation performed on the date correction control 804 is detected, a date option corresponding to July 12 is moved to the area 804A; and after an operation (for example, a tap operation) performed on the confirmation control 805 is detected, the smart band 100 updates the calibrated last menstrual day (for example, July 11) of the latest menstrual period to a date (for example, July 12) currently displayed in the area 804A, and display a calendar 806 of the physiological cycle. The calendar 806 of the physiological cycle indicates the last menstrual day corrected by the user.

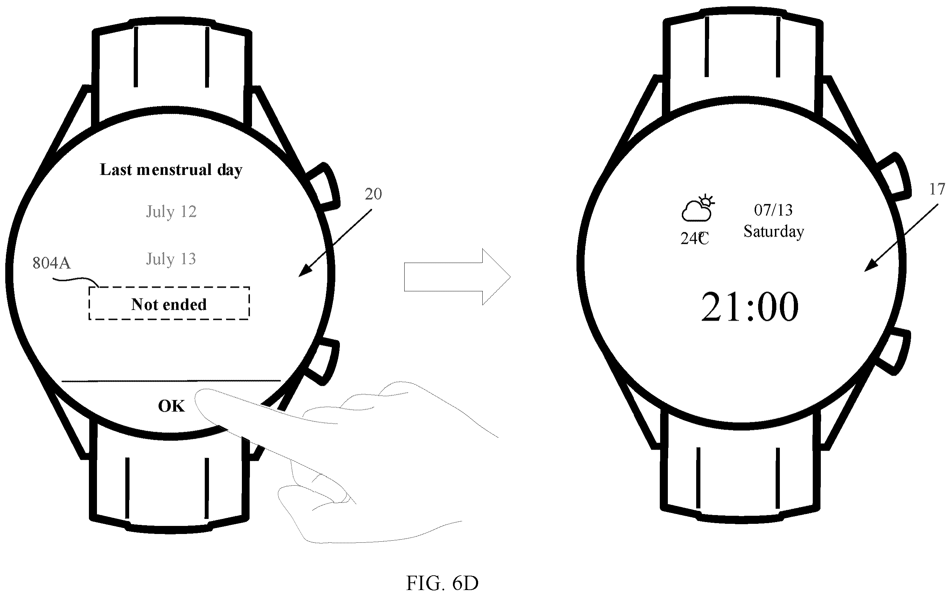

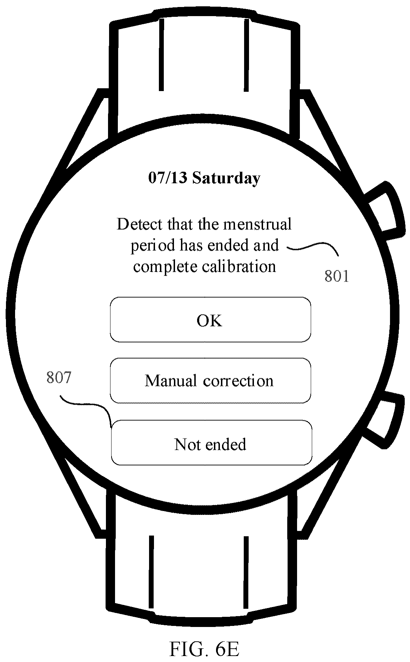

In some embodiments, with reference to FIG. 6B, the date correction control 804 may further include an option 804C used to indicate that the menstrual period does not end, and the option 804C may be used to cancel the calibrated last menstrual day of the latest menstrual period. With reference to FIG. 6B and FIG. 6D, when an upward slide operation performed on the date correction control 804 is detected, the option 804C is moved to the area 804A; and after an operation (for example, a tap operation) performed on the confirmation control 805 is detected, the smart band 100 cancels the calibrated last menstrual day (for example, July 11) of the latest menstrual period. In some embodiments, the option 804C used to indicate that the menstrual period does not end does not need to be displayed in the date correction control 804. As shown in FIG. 6E, a control 807 used to indicate that the menstrual period does not end may be directly displayed along with the last-day prompt information. After detecting an operation (for example, a tap operation) performed by the user on the control 807, the smart band 100 directly cancels the calibrated last menstrual period of the latest menstrual period. It may be understood that, after the user feeds back that the menstrual period does not end, the last day of the current menstrual period continues to be detected, and a reminder is sent again after the last menstrual day is detected.

It should be noted that, in embodiments of this application, the foregoing prompt information (for example, the advance prompt information, the first-day prompt information, or the last-day prompt information) may be displayed in one or more forms such as a text, a picture, an animation, or a video. Not limited to a display, the smart band 100 may alternatively output the foregoing prompt information by using an audio output apparatus (for example, a speaker or a Bluetooth headset). This is not specifically limited herein. An operation of correcting the first/last menstrual day and an operation of canceling the calibrated first/last menstrual day are not specifically limited in embodiments of this application. FIG. 4A to FIG. 6E are examples provided in embodiments of this application, and should not constitute a limitation on this application. In embodiments of this application, the electronic device 200 may also output the foregoing prompt information, and the foregoing prompt information in the electronic device 200 may be presented as a notification message in a notification bar, an MMS message, information in a user interface of the Health app, or the like.

With reference to the foregoing communication system 10, hardware structure, and application scenario, the following describes in detail a menstrual period management method provided in embodiments of this application.

For example, FIG. 7A-1 to FIG. 7A-3 show a method procedure of the menstrual period management method in the solution, and FIG. 7B-1 and FIG. 7B-2 show a specific embodiment of the menstrual period management method. An example in which a detection apparatus includes the smart band 100 is used. The method procedure is applied to the smart band 100 and/or the electronic device 200. In a subsequent embodiment, the electronic device 200 is used as an example for description. The method procedure includes some or all of steps S101 to S119.

S101: Determine a menstrual period trend value 1 of a current date based on physiological feature data of K types of physiological features on last N days, where K is a positive integer.

In this embodiment of this application, a menstrual period trend value of a day indicates a degree to which the day approaches a menstrual peak. In some embodiments, the electronic device 200 determines, on every day, a menstrual period trend value of a current date (for example, a date c) based on the obtained physiological feature data of the K types of physiological features on the last N days. In an implementation, the last N days include the date c. In an implementation, the last N days do not include the date c.

In some embodiments, sensor data collected by a physiological sensor in the detection apparatus (for example, the smart band 100) may be used to obtain physiological data (for example, heart rate data, body temperature data, and heart rate variability indicator data) of the user. The electronic device 200 obtains K types of physiological features of the user on the date c based on physiological data on the date c. For example, the sensor may include some or all of the following: a heart rate sensor, a body temperature sensor, a bone conduction sensor, a pulse sensor, and the like. The K types of physiological features may include some or all of the following indicators: a body temperature, a heart rate, and a heart rate variability.

The heart rate variability (heart rate variability, HRV) refers to a variation of successive heartbeat period differences. One or more indicators of the heart rate variability may include a time domain indicator, for example, a standard deviation of sinus heartbeat NN intervals (standard deviation of NN intervals, SDNN) SDNN, a standard deviation average of sinus heartbeat NN intervals SDANN, and/or a root mean square of successive normal heartbeat period differences (root mean square of successive differences, RMSSD), and may further include a frequency domain indicator, for example, total energy TP, a low frequency power LF, and/or a high frequency power HF. In subsequent embodiments, the SDNN is used as an example to describe the heart rate variability indicator.

In some embodiments, K types of physiological features of the user on the date c are obtained based on physiological data of the user in a resting state on the date c. The resting state may be a state in which the user is in a relatively static state and is not affected by factors such as a muscle activity and mental tension. Whether the user enters the resting state may be detected by using the physiological sensor and/or the motion sensor (for example, an acceleration sensor or a gyro sensor). In some embodiments, the resting state on the date c may include a sleep state of the user (for example, all sleep states on the date c, a deep sleep state on the date c, or a night sleep state on the date c). Whether the user enters the sleep state may be detected by using the physiological sensor and/or the motion sensor. A physiological feature of the user on the date c is determined based on physiological data collected in the sleep state. The foregoing physiological feature in the resting state may also be referred to as a resting body temperature, a resting heart rate, a resting heart rate variability, or the like. Specifically, for how to detect whether the user enters the resting state (for example, the sleep state), refer to an existing implementation. This is not specifically limited in embodiments of this application.

In some embodiments, on the date c, a value of a physiological feature 1 (for example, a heart rate, a body temperature, or a heart rate variability) of the user is equal to an average value of physiological data of the physiological feature 1 in the resting state collected on the date c.

In some embodiments, determining the menstrual period trend value of the current date (for example, the date c) based on the obtained physiological feature data of the K types of physiological features on the last N days in step S101 includes: obtaining the menstrual period trend value on the date c by using the physiological feature data of the K types of physiological features on the last N days and a trained learning model 1. Specifically, in an implementation, a physiological feature sequence matrix of K rows and N columns is generated based on the physiological feature data of the K types of physiological features on the last N days; the physiological feature sequence matrix is input into the trained learning model 1; and the learning model 1 outputs the menstrual period trend value of the date c.

In some embodiments, the learning model is a conventional linear regression model, or a machine learning model or a deep learning model based on a module such as a long short-term memory (Long Short-Term Memory, LSTM) network or a gate recurrent unit (Gate Recurrent Unit, GRU). In this embodiment of this application, physiological feature data of a plurality of historical physiological cycles of the user may be recorded, and the learning model 1 is trained by using the physiological cycle data of the user. In this way, a menstrual period trend value output by the trained learning model 1 matches the physiological feature of the user. For example, FIG. 8 shows an LSTM-based learning model 1. An input layer network of the learning model 1 includes N LSTM units, and an i^{th} LSTM unit in the N LSTM units is configured to input an i^{th} column in the physiological feature sequence matrix with K rows and N columns.

In some embodiments, before the menstrual period trend value of the date c is obtained by using the trained learning model 1, the obtained physiological feature data on the last N days is preprocessed for each physiological feature. The preprocessing includes one or more of band-pass filtering, outlier removal, and interpolation. The band-pass filtering is used to remove interference data, the outlier removal is used to remove data that does not conform to a physiological feature value range, and the interpolation processing is used to supplement undetected missing data or replace an outlier. Then, the menstrual period trend value of the date c is obtained by using the preprocessed physiological feature data of the K types of physiological features on the last N days and the trained learning model 1.

The following describes a historical menstrual period trend sequence and a historical physiological feature sequence in subsequent embodiments.

In some embodiments, after a physiological cycle ends, the historical menstrual period trend sequence is updated based on a menstrual period trend value detected in the physiological cycle. The historical menstrual period trend sequence indicates an average length of historical physiological cycles obtained through comprehensive statistics collection and a daily menstrual period trend value in the historical physiological cycle. The historical menstrual period trend sequence may further indicate a location of a menstrual period in the historical physiological cycle, that is, locations of a first menstrual day and a last menstrual day in the historical physiological cycle. In an implementation, after it is detected that a physiological cycle ends, the historical physiological feature sequence is further updated based on a physiological feature sequence detected in the physiological cycle. Optionally, a length of the historical physiological feature sequence is the same as a length of the historical menstrual period trend sequence, and the historical physiological feature sequence indicates a physiological feature value of each day in a historical physiological cycle obtained through comprehensive statistics collection. In some embodiments, the historical menstrual period trend curve shown in FIG. 3E is obtained after interpolation processing is performed on the historical menstrual period trend sequence, and the historical physiological feature curves shown in FIG. 3E and FIG. 3F are obtained after interpolation processing is performed on the historical physiological feature sequence.

It may be understood that the historical menstrual period trend sequence is a discrete sequence that is obtained through comprehensive statistics collection based on related data of the historical physiological cycle of the user and that can represent a change trend of a menstrual period trend value of the current user in a physiological cycle. The historical physiological feature sequence is a discrete sequence that is obtained through comprehensive statistics collection based on related data of the historical physiological cycle of the user and that can represent a change trend of a physiological feature value of the current user in a physiological cycle.

In an implementation, the first day of the historical physiological cycle obtained through statistics collection is the first menstrual day, and the last day is a day before a next first menstrual day. For example, with reference to a historical menstrual period trend curve shown in (a) in FIG. 9, the curve is generated based on a historical menstrual period trend sequence, a length P1 of a historical physiological cycle indicated by the historical menstrual period trend curve is 28 days, the first day is a first menstrual day, the last day (that is, the 28^{th} day) is a day before a next first menstrual day, and a menstrual period length is 6 days. In an implementation, the collected menstrual period trend values of the first day and the last day of the historical physiological cycle are lowest points in one physiological cycle, the menstrual period is located in a middle location of the historical physiological cycle, and a corresponding menstrual period trend value includes a highest point of the menstrual period trend value. For example, with reference to a historical menstrual period trend curve shown in (b) in FIG. 9, a length P1 of a historical physiological cycle indicated by the historical menstrual period trend curve is 28 days, and menstrual period trend values of the first day and the 28^{th} day are the smallest. A menstrual period is the 11^{th} to the 17^{th} days of the historical physiological cycle. With reference to FIG. 9, it can be learned that, generally, a date farther from the menstrual period indicates a smaller corresponding menstrual period trend value, and a menstrual period trend value in the middle of the menstrual period (for example, the 14^{th} day) is the largest.

The location of the menstrual period in the historical physiological cycle is not specifically limited in this embodiment of this application. (a) and (b) in FIG. 9 are some examples of the historical menstrual period trend sequence provided in this embodiment of this application, and should not constitute a limitation on this embodiment of this application.

In some embodiments, the historical menstrual period trend sequence is updated based on the detected menstrual period trend sequences of last J (for example, 12) historical physiological cycles. A menstrual period trend sequence of a physiological cycle includes a menstrual period trend value corresponding to each day in the physiological cycle. In some embodiments, the historical physiological feature sequence is updated based on detected physiological feature sequences of the last J historical physiological cycles. A physiological feature sequence of a physiological cycle includes a physiological feature value corresponding to each day in the physiological cycle.

In some embodiments, the menstrual period trend sequences of the last J (for example, 12) historical physiological cycles are input into a trained learning model 4, and the learning model 4 outputs the historical menstrual period trend sequence. In an implementation, the menstrual period trend sequences and the physiological feature sequences of the last J (for example, 12) historical physiological cycles are input into a trained learning model 5, and the learning model 5 outputs the historical menstrual period trend sequence and the historical physiological feature sequence.

In some embodiments, after the historical menstrual period trend sequence is obtained, a menstrual period range in the historical physiological cycle obtained after comprehensive statistics collection is determined based on a menstrual period trend value of each day in the historical menstrual period trend sequence. For example, in the historical menstrual period trend sequence, the menstrual period trend value continuously increases for w (for example, 5) days, and a point at which the menstrual period trend value is greater than a first-day threshold (for example, 0.85) for the first time in the w days corresponds to a first menstrual day. In the historical menstrual period trend sequence, the menstrual period trend value continuously decreases for u (for example, 5) days, and a point at which the menstrual period trend value is less than a last-day threshold (for example, 0.85) for the first time in the u days corresponds to a last menstrual day.

In some embodiments, the menstrual period trend sequences of the last J (for example, 12) historical physiological cycles and a calibrated menstrual period range are input into the trained learning model 4, and the learning model 4 outputs the historical menstrual period trend sequence and a menstrual period range in the historical physiological cycle obtained after comprehensive statistics collection. The menstrual period range refers to a date range/time range from a first menstrual day to a last menstrual day of a menstrual period. In an implementation, the menstrual period trend sequences of the last J (for example, 12) historical physiological cycles, the physiological feature sequences, and a calibrated menstrual period range are input into the trained learning model 5, and the learning model 5 outputs the historical menstrual period trend sequence, the historical physiological feature sequence, and a menstrual period range of the historical physiological cycle obtained after comprehensive statistics collection.

In some embodiments, an average value of lengths of the last J historical physiological cycles is obtained, and a physiological cycle length (referred to as a period length 1) corresponding to the historical menstrual period trend sequence is equal to a value obtained after the average value is rounded off; a historical menstrual period trend sequence whose length is the period length 1 is generated based on the menstrual period trend sequences of the last J (for example, 12) historical physiological cycles; and a historical physiological feature sequence whose length is the period length 1 is generated based on the physiological feature sequences of the last J historical physiological cycles.

In an implementation, generating the historical menstrual period trend sequence whose length is the period length 1 based on the menstrual period trend sequences of the last J (for example, 12) historical physiological cycles includes: performing interpolation on a sequence whose sequence length is less than the period length 1 in the J menstrual period trend sequences, where a sequence length after interpolation is equal to the period length 1, a location of an interpolation point is adjacent to a point corresponding to a minimum trend value in the menstrual period trend sequence, and a value of the interpolation point is the minimum value/0. After the interpolation processing, a menstrual period trend sequence 1 whose sequence length is the period length 1 is randomly selected, the menstrual period trend sequence 1 is used as a reference sequence, and other J-1 sequences are respectively a menstrual period trend sequence 2 to a menstrual period trend sequence J. Time domain alignment is performed on another menstrual period trend sequences (for example, the menstrual period trend sequence 2) and the menstrual period trend sequence 1 by using a preset algorithm 3. After alignment, the a^{th} point in the menstrual period trend sequence 1 corresponds to one or more consecutive points in the menstrual period trend sequence 2, and an average value of trend values corresponding to the one or more consecutive points is determined as a value Pₐ₂ of a point in the menstrual period trend sequence 2 corresponding to the a^{th} point. An average value of a value Pₐ₁ of the a^{th} point and values Pₐᵢ of points in the other J-1 trend sequences corresponding to the a^{th} point is obtained, where i is greater than 2 and less than or equal to J, and is used as a value of the a^{th} point in the historical menstrual period trend sequence.

In an implementation, generating the historical physiological feature sequence whose length is the period length 1 based on the physiological feature sequences of the last J historical physiological cycles includes: performing interpolation on a sequence whose sequence length is less than the period length 1 in the J physiological feature sequences (for example, the physiological feature sequence 1, where a historical physiological cycle of the physiological feature sequence 1 corresponds to the menstrual period trend sequence 1), where a sequence length after interpolation is equal to the period length 1, a location of an interpolation point is the same as an interpolation location in the menstrual period trend sequence 1, and a value of the interpolation point is an average value of two adjacent points. After interpolation processing, a physiological feature sequence trend sequence 1 whose sequence length is the period length 1 is randomly selected, the physiological feature sequence trend sequence 1 is used as a reference sequence, and the other J-1 sequences are respectively a physiological feature sequence trend sequence 2 to a physiological feature trend sequence J. Time domain alignment is performed on another physiological feature sequence (for example, the menstrual period trend sequence 2) and the physiological feature trend sequence 1 by using a preset algorithm 3. After alignment, the a^{th} point in the physiological feature trend sequence 1 corresponds to one or more consecutive points in the physiological feature sequence 2, and an average value of physiological feature values corresponding to the one or more consecutive points is determined as a value Pₐ₂ of a point in the physiological feature sequence 2 corresponding to the a^{th} point. An average value of a value Pₐ₁ of the a^{th} point and values Pₐᵢ of points in the other J-1 physiological feature sequences corresponding to the a^{th} point is obtained, where i is greater than 1 and less than or equal to J, and is used as a value of the a^{th} point in the historical physiological feature sequence.

Not limited to the foregoing implementation of collecting statistics on the historical menstrual period trend sequence and the historical physiological feature sequence, the historical menstrual period trend sequence and the historical physiological feature sequence may alternatively be obtained in another implementation. This is not specifically limited herein.

In some embodiments, the historical menstrual period trend sequence is updated based on detected menstrual period trend sequences of all historical physiological cycles of the current user. In some embodiments, the historical physiological feature sequence is updated based on detected physiological feature sequences of all the historical physiological cycles.

The menstrual period trend curve shown in FIG. 9 is a continuous historical menstrual period trend curve generated after interpolation processing is performed on a discrete menstrual period trend sequence. Similarly, after interpolation processing is performed on the historical physiological feature sequence, a continuous historical physiological feature curve may be obtained, for example, historical physiological feature curves described in FIG. 3E and FIG. 3F.

S102: Perform anomaly detection based on the menstrual period trend value 1 and/or the physiological feature of the current date; and if an anomaly exists, indicate an abnormal situation.

As shown in FIG. 7B-1 and FIG. 7B-2, step S102 includes: S102A: Perform anomaly detection to determine whether an abnormal situation exists; and if an abnormal situation exists, perform S102B; or if no abnormal situation exists, perform S103; and S102B: indicate the abnormal situation.

In this embodiment of this application, before a menstrual period prediction result (for example, a predicted menstrual period displayed by using the calendar 201 of the physiological cycle shown in FIG. 3D, and a predicted future menstrual period trend value displayed by using the trend value curve 301 shown in FIG. 3E) is displayed to the user based on the detected menstrual period trend value, and intelligent menstrual period calibration is performed, one or more of the menstrual period trend value of the current date c, the physiological feature of the date c, the historical menstrual period trend value, and the historical physiological feature are used together to detect a possible abnormal situation. The abnormal situation includes but is not limited to the following four abnormal situations:
Abnormal situation 1: The user is changed.

Specifically, whether the current user wearing the detection apparatus (for example, the smart band 100) changes is detected. In some embodiments, one or more pieces of physiological feature data of the current user are obtained, and any one of the foregoing one or more pieces of physiological feature data is comprehensively compared with a historical indicator of the feature of a historical user, to determine whether the abnormal situation 1 is detected. In an implementation, on the date c, if there are at least M pieces of physiological feature data that are statistically significantly contrary to the historical indicator of the historical user (that is, at least M pieces of physiological feature data are detected to be abnormal), it is determined that the abnormal situation 1 is detected. M is equal to 1, or M (for example, 2) is greater than 1 and less than a total quantity (for example, 3) of physiological feature data, or M is equal to a total quantity of physiological feature data.

In some embodiments, the one or more pieces of physiological feature data include some or all of menstrual period trend values of last H1 days, physiological feature data of last H2 days, and physiological change trends of last H3 days. The foregoing last H1 days, last H2 days, and last H3 days all include the date c, where H1, H2, and H3 are positive integers. H1, H2, and H3 may be equal, or may not be equal. This is not specifically limited herein. For example, H1, H2, and H3 are all equal to 28.

In an implementation, the historical indicator of the historical user corresponding to the menstrual period trend value of the last H1 days includes a historical menstrual period trend sequence. The historical menstrual period trend sequence indicates a daily menstrual period trend value of the user in a physiological cycle obtained after comprehensive statistics collection. The physiological cycle of the historical user obtained after comprehensive statistics collection has P1 days, and the historical menstrual period trend sequence includes P1 discrete points. Comparing the menstrual period trend values of the last H1 days with the historical menstrual period trend sequence includes: obtaining a sequence 1 corresponding to the obtained menstrual period trend values of the last H1 days; performing time domain alignment between the sequence 1 and the historical menstrual period trend sequence by using a preset algorithm 1, so as to determine a sequence segment 1 corresponding to the sequence 1 in the historical menstrual period trend sequence, where the segment includes H1 consecutive discrete points; and determining a similarity between the sequence 1 and the sequence segment 1 by using a preset algorithm 2. If the similarity is less than a similarity threshold, the physiological feature data (that is, the menstrual period trend values of the last H1 day) is abnormal.

The preset algorithm 1 and the preset algorithm 2 are not specifically limited in this embodiment of this application. For example, the preset algorithm 1 is a sliding window crosscorrelation algorithm, a subsequence matching algorithm, a dynamic time warping (dynamic time warping, DTW) algorithm, or the like, and the preset algorithm 2 is a Pearson correlation coefficient algorithm, a Euclidean distance algorithm, a Cosine similarity algorithm, or the like. H1 may be equal to the quantity P1 of days (for example, 28 days) of the historical physiological cycle of the historical user in statistics, or may be less than P1 (for example, 7 days). This is not specifically limited herein.

In an implementation, the historical indicator of the historical user corresponding to the physiological features of the last H2 days includes a historical physiological feature sequence. The historical physiological feature sequence is obtained for each physiological feature (for example, a body temperature, a heart rate, or a heart rate variability). The historical physiological feature sequence indicates a daily physiological feature value of the historical user in a physiological cycle in statistics. The historical physiological feature sequence includes P1 discrete points. Comparing the physiological feature data of the last H2 days with the historical physiological feature sequence includes: for each physiological feature, performing, by using a preset algorithm 1, time domain alignment between a sequence 2 corresponding to the physiological feature data of the last H2 days and the historical physiological feature sequence, to determine a sequence segment 2 that is in the historical physiological feature sequence and that is corresponding to the sequence 2, where the segment includes H2 consecutive discrete points; and determining a similarity between the sequence 2 and the sequence segment 2 by using a preset algorithm 2 after the alignment. If a similarity corresponding to at least one or all physiological features is less than a similarity threshold, the physiological feature data (that is, the physiological feature data of the last H2 days) is abnormal. H2 may be equal to P1 (for example, 28 days), or may be less than P1 (for example, 7 days). This is not specifically limited herein.

In an implementation, the historical indicator of the historical user corresponding to the physiological change trends of the last H3 days includes a standard change trend sequence; and a historical change trend sequence is obtained for each physiological feature (for example, a body temperature, a heart rate, or a heart rate variability), where a value of an (i-1)^{th} point in the historical change trend sequence indicates a difference between a physiological feature of an i^{th} point and a physiological feature of an (i-1)^{th} point in the historical physiological feature sequence, and i is a positive integer greater than or equal to 2. The historical change trend sequence includes P1-1 discrete points. Comparing the physiological change trends of the last H3 days with the historical change trend sequence includes: obtaining, for each physiological feature, a sequence 3 of a change trend corresponding to the physiological feature data of the last H3 days, where a value of a (j-1)^{th} point in the sequence 3 indicates a difference between physiological features of a j^{th} day and a (j-1)^{th} day in the last H3 days, and j is a positive integer greater than or equal to 2. It may be understood that the change trend sequence 1 includes H3-1 discrete points, and a value 1 of the last point is a difference between a physiological feature of the date c and a physiological feature of the day before the date c. Time domain alignment is performed between the sequence 3 and the historical change trend sequence by using a preset algorithm 1, to determine a value 2 of a point that is in the historical change trend sequence and that is corresponding to the last point of the sequence 3. A difference 1 between the value 1 and the value 2 is determined. If the difference 1 corresponding to at least one or all physiological features is greater than a preset change threshold, the physiological feature data (that is, the physiological change trends of the last H3 days) is abnormal. H3 may be equal to P1 (for example, 28 days), or may be less than P1 (for example, 7 days). This is not specifically limited herein.

In an implementation, when the abnormal situation 1 is detected, the user is prompted with the abnormal situation 1 on the date c. In an implementation, the abnormal situation 1 is detected, and the date c is marked with a mark 1 (that is, a correspondence between the date c and the mark 1 is recorded), where the mark 1 indicates that the user changes; and the abnormal situation 1 is prompted to the user only after V consecutive days (for example, three days) are marked with the mark 1.

In some embodiments, prompting the user with the abnormal situation 1 includes: outputting prompt information 3, where the prompt information 3 indicates to determine whether to change the user when it is detected that the user of the smart band 100 is changed. After determining to change the user, the electronic device 200 no longer performs menstrual period management based on the historical menstrual period trend sequence and the historical physiological feature sequence of the historical user, but registers a new user, and re-collects statistics on the historical menstrual period trend sequence and the historical physiological feature sequence for the new user, so as to generate a new user profile for the new user. The electronic device 200 may directly display the prompt information 3 by using a notification message in a notification center, or may pop up the prompt information 3 after the user starts the Health app.

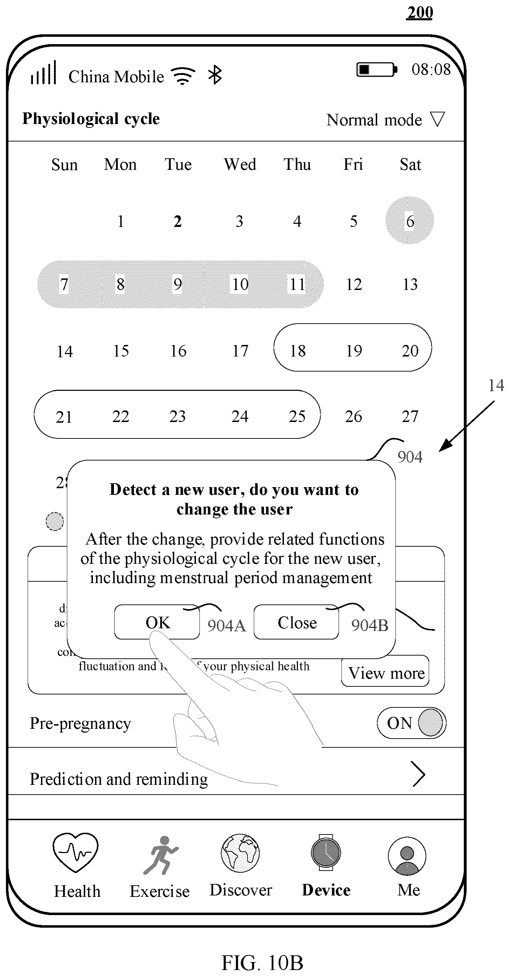

For example, with reference to FIG. 10A, after the abnormal situation 1 is detected, a notification message 903 is displayed in a notification center interface 21, and the notification message 903 includes the foregoing prompt information 3. As shown in FIG. 10B, after detecting that the user taps the notification message 903, the electronic device 200 opens a user interface 14 of a physiological cycle of the Health app, and displays a prompt box 904. The prompt box 904 includes the foregoing prompt information 3, and may further include a confirmation control 904A and a close control 904B. The close control 904B is used to close the prompt box 904. After it is detected that the user taps the confirmation control 904A, the user of the physiological cycle service is switched to a new user. For example, as shown in FIG. 10B, after it is detected that the user taps the confirmation control 904A, a user management interface 22 may be further displayed. The user management interface 22 includes a basic information editing box 905 of the new user. For example, the basic information includes a nickname, a height, a weight, an age, a menstrual period length, and a cycle length. The menstrual period length and the cycle length that are set by the user are used to predict a menstrual period by using the foregoing conventional calendar method at an initial stage of providing menstrual period management for the new user; and after a menstrual period trend value and a physiological feature of at least one physiological cycle of the new user are collected, the menstrual period management method provided in this embodiment of this application is used to perform intelligent menstrual period prediction, menstrual period reminding, and menstrual period re-calibration.

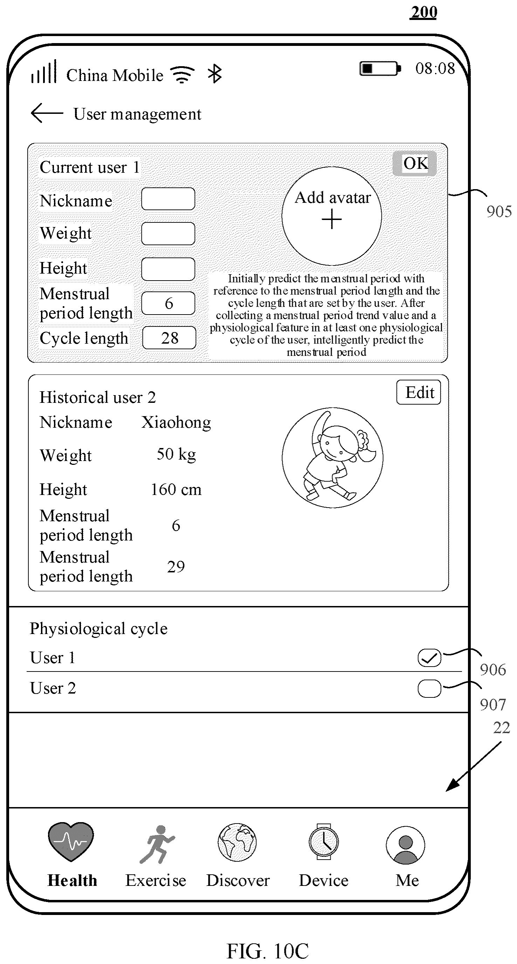

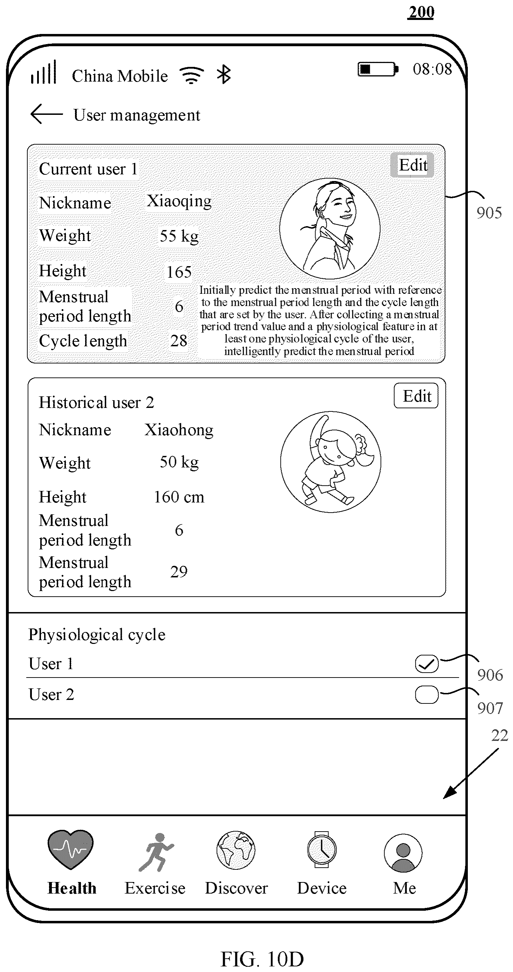

For example, as shown in FIG. 10C, when the user management interface 22 includes a plurality of users, the user management interface 22 may further include an option of each user corresponding to the physiological cycle, for example, an option 906 of a user 1 (that is, a new user) and an option 907 of a user 2 (that is, a historical user). Only one of options of users is in a selected state. For example, the option 906 of the user 1 is currently in a selected state, and the selected user is a current user of the physiological cycle function service. The user can manually switch the option of the currently selected user. After the user is changed this time, if the user is switched back to the user 2, to provide menstrual period management for the user 2 in a timely manner, the user may tap the option 907 of the user 2. In response to a tap operation of the user, the electronic device 200 may switch the option 907 of the user 2 to a selected state, and the option 906 of the user 1 to an unselected state, that is, switch the current user to the user 2. As shown in FIG. 10D, after editing the basic information of the new user, the user may return to the user interface 14 of the physiological cycle, and the calendar 201 in the user interface 14 is used to display the physiological cycle of the new user.

In some embodiments, after the user is changed, the electronic device 200 may remind the user whether to delete related data of the historical user, or may automatically delete related data of the historical user.

Abnormal situation 2: The current user is not a female.

Specifically, whether the current user wearing the detection apparatus (for example, the smart band 100) is a female user is detected. In some embodiments, one or more pieces of physiological feature data (for example, menstrual period trend values of last H1 days) of the current user are obtained, and any one of the foregoing one or more pieces of physiological feature data is comprehensively compared with a female standard indicator corresponding to the feature in statistics, so as to determine whether the abnormal situation 2 is detected. In an implementation, if at least M pieces of physiological feature data significantly contradict the female standard indicator on the date c, that is, at least M pieces of physiological feature data are detected to be abnormal, it is determined that the abnormal situation 2 is detected. M is equal to 1, or M (for example, 2) is greater than 1 and less than a total quantity (for example, 3) of physiological feature data, or M is equal to a total quantity of physiological feature data. In an implementation, if all physiological feature data of the date c significantly contradicts the female standard indicator, it is determined that the abnormal situation 2 is detected.

In an implementation, the female standard indicator corresponding to the menstrual period trend values of the last H1 days includes a statistical standard menstrual period trend sequence of a female. The standard menstrual period trend sequence indicates a standard menstrual period trend value of each day in a physiological cycle. The statistical standard physiological cycle has P2 days (for example, 28), and the standard menstrual period trend sequence includes P2 discrete points. For an implementation of comparing the menstrual period trend values of the last H1 days with the standard menstrual period trend sequence, refer to the implementation of comparing the menstrual period trend values of the last H1 days with the historical menstrual period trend sequence. Details are not described herein again.

In an implementation, the female standard indicator corresponding to the physiological features of the last H2 days includes a standard physiological feature sequence. A standard physiological feature sequence is obtained for each physiological feature (for example, a body temperature, a heart rate, or a heart rate variability). The standard physiological feature sequence indicates a standard physiological feature value of each day in a physiological cycle in statistics. The standard physiological feature sequence includes P2 discrete points. For an implementation of comparing the physiological feature data of the last H2 days with the standard physiological feature sequence, refer to the implementation of comparing the physiological feature data of the last H2 days with the historical physiological feature sequence. Details are not described herein again.

In an implementation, the female standard indicator corresponding to the physiological change trends of the last H3 days includes a standard change trend sequence; and a standard change trend sequence is obtained for each physiological feature (for example, a body temperature, a heart rate, or a heart rate variability), where a value of an (i-1)^{th} point in the standard change trend sequence indicates a difference between a physiological feature of an i^{th} point and an (i-1)^{th} point in the standard physiological feature sequence, and i is a positive integer greater than or equal to 2. The standard change trend sequence includes P2-1 discrete points. For an implementation of comparing the physiological change trends of the last H3 days with the standard change trend sequence, refer to the implementation of comparing the physiological change trends of the last H3 days with the historical change trend sequence. Details are not described herein again.

Similar to the abnormal situation 1, the abnormal situation 2 may be prompted to the user on the date c after the abnormal situation 2 is detected, or the abnormal situation 2 may be prompted to the user only after the abnormal situation 2 is marked for V consecutive days (for example, 3 days).

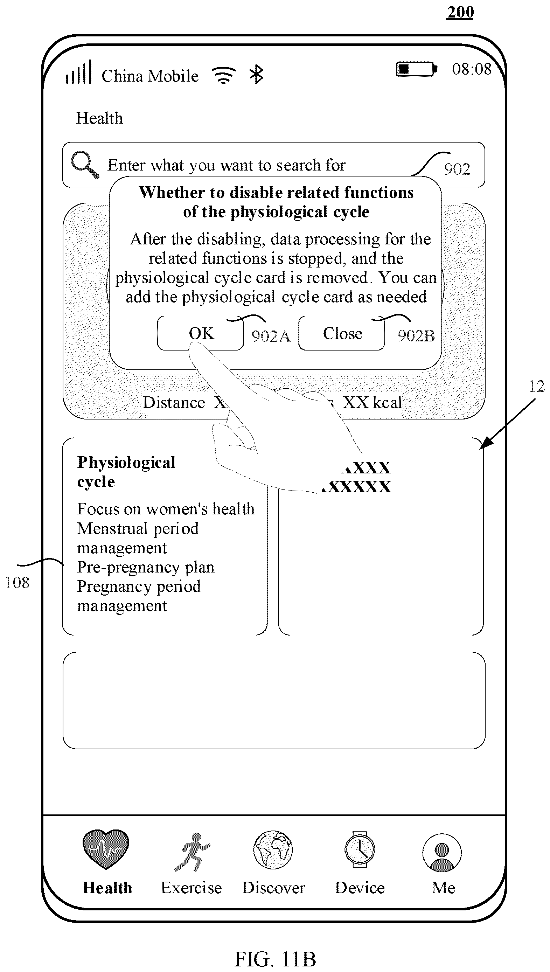

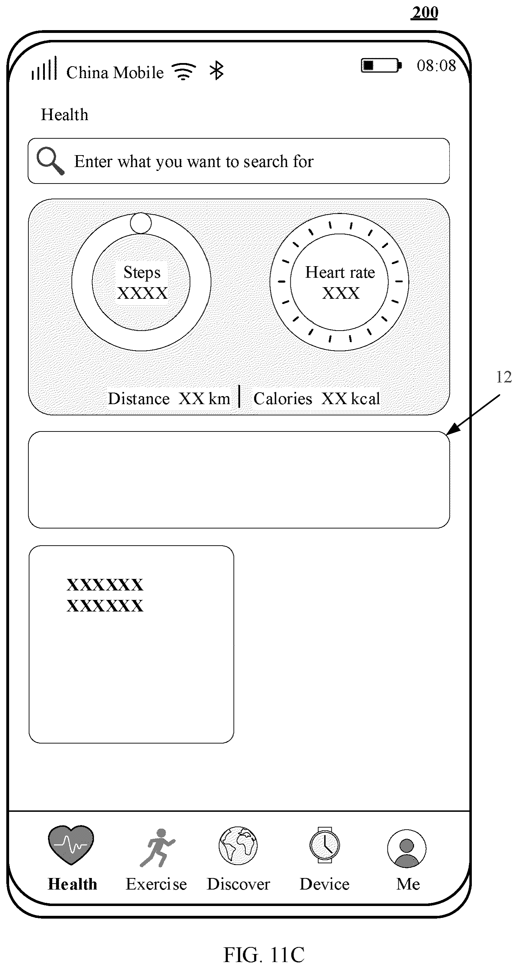

In some embodiments, prompting the user with the abnormal situation 2 includes: outputting prompt information 2, where the prompt information 2 indicates whether a function related to the physiological cycle needs to be disabled, including menstrual period management. The electronic device 200 may directly display the prompt information 2 by using a notification message in a notification center, or may pop up the prompt information 2 after the user starts the Health app. For example, as shown in FIG. 11A, the electronic device 200 displays a Health notification message 901 in a notification center interface 21, and the notification message 901 includes the foregoing prompt information 2. As shown in FIG. 11B, after detecting that the user taps the notification message 901, the electronic device 200 opens a user interface 12 of the Health app, and displays a prompt box 902. The prompt box 902 includes the foregoing prompt information 2, and may further include a confirmation control 902A and a close control 902B. Content of the prompt information 2 may include "Whether to disable the physiological cycle related function, and if disabled, the physiological cycle card is removed, and may be added when necessary". The close control 902B is used to close the prompt box 902. As shown in FIG. 11C, after it is detected that the user taps the confirmation control 902A, the physiological cycle related function (for example, menstrual period management) is disabled, and the physiological cycle card 108 in the user interface 12 of the Health app is removed.

Abnormal situation 3: The user is pregnant.

Specifically, whether the current user wearing the detection apparatus (for example, the smart band 100) is pregnant is detected. In some embodiments, one or more pieces of physiological feature data of the user are obtained, and input into a trained learning model 2. Output information of the learning model 2 indicates whether the user is pregnant. For the learning model 2, refer to related descriptions of the learning model 1. In some embodiments, one or more pieces of physiological feature data of the user are obtained, and when it is detected that the one or more pieces of physiological feature data meet a pregnancy condition, it is determined that the abnormal situation 3 is detected.

In some embodiments, the one or more pieces of physiological feature data include some or all of menstrual period trend values of last T1 days, physiological feature data of last T2 days, and physiological change trends of last T3 days. The last T1 days, the last T2 days, and the last T3 days all include the date c, where T1, T2, and T3 are positive integers. T1, T2, and T3 may be equal or may not be equal. This is not specifically limited herein. For example, T1, T2, and T3 are all equal to 18.

In an implementation, the one or more pieces of physiological feature data include menstrual period trend values of the user on the last T1 days and body temperatures on the last T2 days. The pregnancy condition includes: menstrual period trend values of the last T1 (for example, 18) days are all at a low level, that is, all less than a preset trend value 1 (for example, 0.3), and/or body temperatures on the last T2 days are all high, that is, all greater than a preset temperature 1 (for example, 36.8).

In an implementation, the one or more pieces of physiological feature data include the menstrual period trend values of the last T1 days and the physiological feature data of the last T2 days. In an implementation, for each physiological feature, a statistical physiological feature sequence of a pregnancy period in an i^{th} month of pregnancy is obtained, where a value of i is a positive integer from 1 to 10. For each physiological feature, physiological feature data of the last T2 days is compared with the physiological feature sequence of the pregnancy period in the i^{th} month, to determine a similarity of the i^{th} month corresponding to the physiological feature; and a maximum value of similarities in 10 months is determined as the similarity corresponding to the physiological feature. The pregnancy condition includes that a similarity corresponding to each physiological feature is greater than a preset threshold, and menstrual period trend values of the last T1 days are all at a low level. For how to compare the physiological feature data of the last T2 days with the physiological feature sequence of the pregnancy period in the i^{th} month, to determine the similarity of the i^{th} month corresponding to the physiological feature, refer to the foregoing implementation of comparing the physiological feature data of the last H2 days with the historical physiological feature sequence. Details are not described herein again.

Similar to the abnormal situation 1, the abnormal situation 3 may be prompted to the user on the date c after the abnormal situation 3 is detected, or the abnormal situation 3 may be prompted to the user only after the abnormal situation 3 is marked for V consecutive days (for example, three days).

In some embodiments, prompting the user with the abnormal situation 3 includes: outputting prompt information 4, where the prompt information 4 indicates whether to switch the management mode of the physiological cycle to the pregnancy mode when it is detected that the user is pregnant; and in the pregnancy mode, a related function of menstrual period management is no longer provided, but a related function of pregnancy period management is provided. The electronic device 200 may directly display the prompt information 4 by using a notification message in a notification center, or may pop up the prompt information 4 after the user starts the Health app.

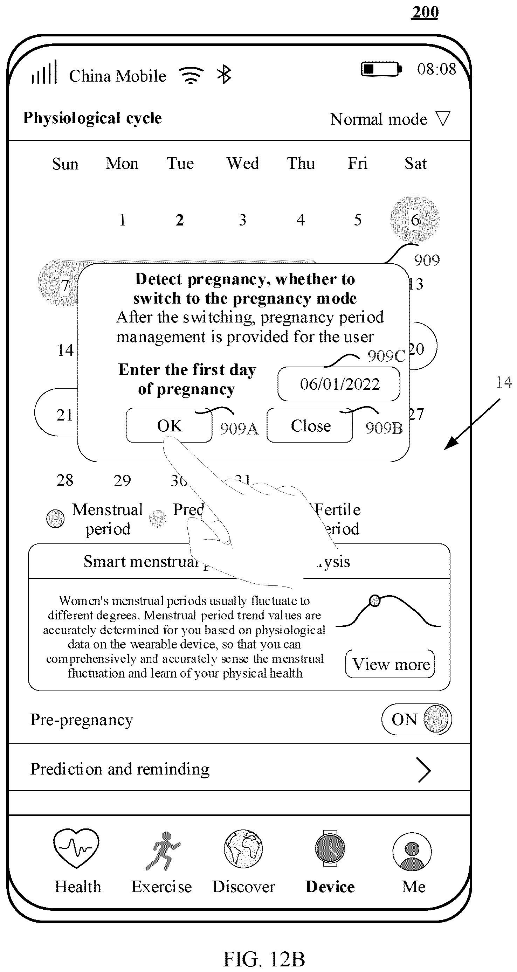

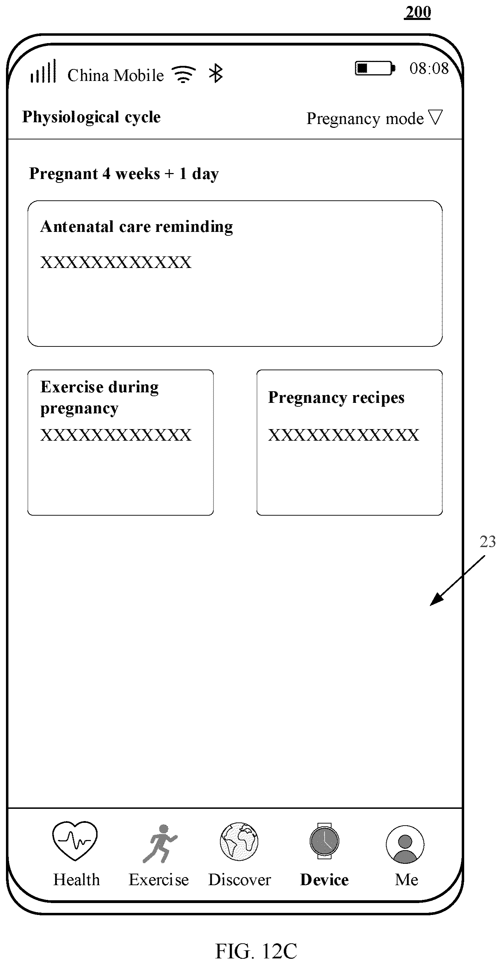

For example, with reference to FIG. 12A, after the abnormal situation 3 is detected, a notification message 908 is displayed in a notification center interface 21, and the notification message 908 includes the foregoing prompt information 4. As shown in FIG. 12B, after detecting that the user taps the notification message 908, the electronic device 200 opens a user interface 14 of a physiological cycle of the Health app, and displays a prompt box 909. The prompt box 909 includes the foregoing prompt information 4, and may further include a confirmation control 909A, a close control 909B, and an input box 909C. The close control 909B is used to close the prompt box 909, and the input box 909C is used to enter a pregnancy date. As shown in FIG. 12C, after it is detected that the user taps the confirmation control 909A, the physiological cycle mode is switched to the pregnancy mode, and a user interface 23 of the pregnancy mode is displayed. On the user interface 23, a related function of pregnancy management is provided for the user with reference to the pregnancy date entered by the user in the input box 909C, for example, predicting and planning an antenatal care time, and recommending recipes and exercise manners in different months of pregnancy.

Abnormal situation 4: The user is sick.

Specifically, whether the current user wearing the detection apparatus (for example, the smart band 100) is sick is detected. In some embodiments, one or more pieces of physiological feature data of the user are obtained, and input into a trained learning model 3. Output information of the learning model 3 indicates whether the user is sick. For the learning model 3, refer to related descriptions of the learning model 1. In some embodiments, one or more pieces of physiological feature data of the user are obtained, and when it is detected that the one or more pieces of physiological feature data meet a sick condition, it is determined that the abnormal situation 4 is detected.

In some embodiments, the one or more pieces of physiological feature data include some or all of menstrual period trend values of last R1 days, physiological feature data of last R2 days, and physiological change trends of last R3 days. The last R1 days, the last R2 days, and the last R3 days all include the date c, where R1, R2, and R3 are positive integers. R1, R2, and R3 may be equal or may not be equal. This is not specifically limited herein. For example, R1, R2, and R3 are all equal to 22.

In some embodiments, the one or more pieces of physiological feature data include menstrual period trend values of the last R1 days, a length of a latest physiological cycle, a length of a historical physiological cycle corresponding to a historical menstrual period trend sequence, and physiological feature data used to detect whether the user is pregnant. The last R1 days include the date c, and R1 is a positive integer. For example, R1 is equal to 22. The sick condition includes: Menstrual period trend values of the last R1 days are all at a low level, no user pregnancy is detected, and a length of a latest physiological cycle is significantly reduced or increased compared with a length of a historical physiological cycle corresponding to a historical menstrual period trend sequence, that is, a difference between the two is greater than a preset quantity of days (for example, 10).

Similar to the abnormal situation 1, the abnormal situation 4 may be prompted to the user on the date c after the abnormal situation 4 is detected, or the abnormal situation 4 may be prompted to the user only after the abnormal situation 4 is marked for V consecutive days (for example, 3 days).

In some embodiments, prompting the user with the abnormal situation 4 includes: outputting prompt information 5, where the prompt information 5 indicates a possible disease. Based on an input operation of the user, after the user is confirmed to be sick, the user is reminded that the menstrual period management function may fail due to the disease. In this case, a menstrual period trend result is only for reference. In some embodiments, the prompt information 5 is further used to prompt the user to upload a related medical check report if the user has confirmed that the user is sick. The electronic device 200 performs optical character recognition (Optical Character Recognition, OCR) information extraction on the medical check report uploaded by the user, and determines, according to the extracted information, whether the user is sick. The electronic device 200 may directly display the prompt information 5 by using a notification message in a notification center, or may pop up the prompt information 5 after the user starts the Health app.

The foregoing implementations of detecting the abnormal situation 1 to the abnormal situation 4 are not limited. Based on the menstrual period trend value and/or the physiological feature, in this embodiment of this application, any one of the abnormal situation 1 to the abnormal situation 4 may be detected in another implementation. This is not specifically limited herein.

In this embodiment of this application, step S102 is optional. In some embodiments, S102 does not need to be performed.

S103: Update menstrual period data 1 based on the menstrual period trend value 1, where the menstrual period data 1 includes a part or all of the following: a menstrual period trend value corresponding to the current date, a future menstrual period trend value predicted based on the menstrual period trend value of the current date, and a first menstrual day, a last menstrual day, and a physical symptom predicted based on the future menstrual period trend value.

In this embodiment of this application, after obtaining the menstrual period trend value 1, the electronic device 200 records the menstrual period trend value corresponding to the date c as the menstrual period trend value 1, and updates the menstrual period trend value corresponding to the date c in the menstrual period trend curve shown in FIG. 3E.

In some embodiments, predicting the future menstrual period trend value based on the menstrual period trend value of the date c includes: determining a sequence 4 corresponding to menstrual period trend values obtained in last C days; and performing time domain alignment between the sequence 4 and the historical menstrual period trend sequence by using a preset algorithm 1, so as to determine a point corresponding to a last point (for example, a point corresponding to the date c) that is in the historical menstrual period trend sequence and that corresponds to the sequence 4, where the point is corresponding to an n^{th} day of a historical physiological cycle. A menstrual period trend value of a future f^{th} day is predicted as a menstrual period trend value corresponding to the [(n+f)%P1]^{th} point in the historical menstrual period trend sequence. For example, with reference to FIG. 13, (a) in FIG. 13 shows a historical menstrual period trend curve corresponding to a historical menstrual period trend sequence; and (a) and (b) in FIG. 13 show a sequence 4 corresponding to menstrual period trend values of last C days. In (a) in FIG. 13, C is equal to a quantity of days from a date a to a date c; and in (b) in FIG. 13, C is equal to a quantity of days of a physiological cycle corresponding to the historical menstrual period trend sequence. As shown in (b) and (c) in FIG. 13, after the sequence 4 is aligned with the historical menstrual period trend sequence, the date c corresponds to the 26^{th} day of the historical menstrual period trend sequence.

In some embodiments, based on the first menstrual day predicted based on the future menstrual period trend value, the historical menstrual period trend sequence indicates a location of the menstrual period in the physiological cycle. For example, the historical menstrual period trend sequence shown in (a) in FIG. 13 indicates that the menstrual period is the first to sixth days of the physiological cycle, and the date c corresponds to the 26^{th} day of the historical menstrual period trend sequence. Therefore, it is predicted that the first day of the next menstrual period is the third day after the date c, and the last day of the next menstrual period is the ninth day after the date c.

For example, with reference to (a) in FIG. 13, it is predicted that a menstrual period trend curve after the date c includes a curve after a point corresponding to the date c in the historical menstrual period trend curve; and a menstrual period trend value curve corresponding to a date that exceeds the current physiological cycle cyclically uses the historical menstrual period trend curve. Then, the menstrual period trend curve after the date c in the menstrual period trend curve shown in FIG. 3E is updated.

In some embodiments, the electronic device 200 records a physical symptom corresponding to each day of the physiological cycle corresponding to the historical menstrual period trend sequence; and after determining a point (for example, the 26^{th} day) corresponding to the date c in the historical menstrual period trend sequence, determines that the physical symptom corresponding to the date c is a physical symptom corresponding to the 26^{th} day. For example, with reference to FIG. 3G, the electronic device 200 updates, in the user interface 16, the physical symptom corresponding to the date c.

S104: When the current date is a date 1, and the menstrual period trend value 1 meets a preset condition 1, predict that a first day of a menstrual period 1 is in X days.

S105: Output advance prompt information, where the advance prompt information indicates that the menstrual period is to start after/within X days.

In some embodiments, the electronic device 200 may remind the user X (for example, 3) days in advance before the first menstrual day after predicting the first menstrual day. For example, with reference to FIG. 4A, the electronic device 200 indicates the smart band 100 to display the advance prompt information, that is, the advance prompt information 601.

In some embodiments, the electronic device 200 may receive an input operation of the user, to enable prediction and reminding before the menstrual period, and set a quantity X of days and/or a reminding timing of advance reminding. With reference to FIG. 3H, the user may enable advance prediction and reminding for the menstrual period by using the switch control 501. The user may set a value of X in advance by using the setting control 504. The user may set, by using the timing option 506, that menstrual period advance reminding is performed on each of X days before the menstrual period, or set, by using the timing option 507, that menstrual period advance reminding is performed on the X^{th} day before the menstrual period.

In some embodiments, with reference to S104 in FIG. 7B-1 and FIG. 7B-2, the preset condition 2 includes that the menstrual period trend value is greater than a threshold 1. In some embodiments, the preset condition 1 includes that a difference between the menstrual period trend value and the threshold 1 is less than a preset value 1, where the preset value 1 is a preset smaller value, for example, 0.05. In some embodiments, the electronic device 200 determines, based on a value of X, a menstrual period trend value Z1 corresponding to the X^{th} day before the menstrual period, where the threshold 1 is equal to Z1. In an implementation, a menstrual period trend value Z1 corresponding to the X^{th} day before the menstrual period is determined based on the historical menstrual period trend sequence.

In some embodiments, time domain alignment is performed between a sequence 5 corresponding to menstrual period trend values of last C days and the historical menstrual period trend sequence. The preset condition 2 includes: after the alignment, a point that is corresponding to the current date c (that is, the date 1) in the sequence 5 and that is in the historical menstrual period trend sequence is a point corresponding to the third day before the menstrual period.

Steps S104 and S105 are optional. In some embodiments, steps S104 and S105 do not need to be performed.

S106: When an input operation 1 is detected, perform correction and calibrate a date 4 as the first menstrual day of the current menstrual period (menstrual period 1).

In some embodiments, if the user sees the advance prompt information, and the menstrual period has arrived, the first menstrual day may be calibrated by using the input operation 1. With reference to FIG. 4B and FIG. 4C, a correction control 603 is further displayed along with the advance prompt information 601. The input operation 1 includes some or all of the following: an input operation (for example, a tap operation) performed on the correction control 603, an input operation (for example, a slide operation for selecting a date 4) performed on the date correction control 604, and an operation (for example, a tap operation) performed on the confirmation control 605. The date correction control 604 is configured to select the first menstrual day, and the confirmation control 605 is configured to confirm the selected date 4.

In some embodiments, with reference to FIG. 4A, a confirmation control 602 is further displayed along with the advance prompt information 601. The confirmation control 602 indicates the user to confirm that the advance prompt information is known.

Steps S104 to S106 are optional. In some embodiments, steps S104 to S106 do not need to be performed.

S107: When the current date is a date 2, and the menstrual period trend value 2 meets a preset condition 2, calibrate the date 2 as the first menstrual day of the current menstrual period (menstrual period 1).

S108: Output first-day prompt information.

In this embodiment of this application, it is determined, based on the menstrual period trend value of the current date, that the current day is the first menstrual day, and the user is reminded by using first-day prompt information. For example, with reference to FIG. 5A, the electronic device 200 indicates the smart band 100 to display first-day prompt information 701. In some embodiments, with reference to FIG. 3H, the user may enable the first day reminder of the menstrual period by using the switch control 502.

In some embodiments, with reference to S107 in FIG. 7B-1 and FIG. 7B-2, the preset condition 2 includes that the menstrual period trend value is greater than the first-day threshold. In some embodiments, the preset condition 2 includes that the menstrual period trend value continuously increases for w (for example, 5) days on the current date and a previous date, and the menstrual period trend value on the current date (for example, the date 2) in the w days is greater than the first-day threshold (for example, 0.85) for the first time.

In some embodiments, with reference to (a) in FIG. 13, time domain alignment is performed between the sequence 5 corresponding to the menstrual period trend value of the last C days and the historical menstrual period trend sequence. The preset condition 1 includes: after the alignment, a point that is corresponding to the current date (that is, the date 2) in the sequence 5 and that is in the historical menstrual period trend sequence is a point corresponding to the first menstrual day.

S109: When an input operation 2 is detected, determine that the calibrated first menstrual day (that is, the date 2) is correct.

In some embodiments, with reference to FIG. 5B and FIG. 5C, a confirmation control 702 is further displayed along with the first-day prompt information 701. If the user determines that the date 2 is the first menstrual day of the menstrual period 1, an input operation (for example, a tap operation) performed on the confirmation control 702 may indicate that the prompted first menstrual day is correct.

S110: When an input operation 3 is detected, perform correction and calibrate a date 5 as the first menstrual day of the menstrual period 1.

In some embodiments, with reference to FIG. 5B and FIG. 5C, a correction control 703 is further displayed along with the first-day prompt information 701. If the menstrual period has arrived but it is detected that the first menstrual day is incorrect, the user may correct the date of the first menstrual day by acting on the correction control 703. The input operation 3 may include some or all of the following: an input operation (for example, a tap operation) performed on the correction control 703, an input operation (for example, a slide operation for selecting a date 5) performed on the date correction control 704, and an operation (for example, a tap operation) performed on the confirmation control 705. The date correction control 704 is configured to select the corrected first menstrual day (that is, the date 5), and the confirmation control 705 is configured to confirm the selected date 5.

S111: When an input operation 4 is detected, cancel calibrating the date 2 as the first menstrual day.

In some embodiments, with reference to FIG. 5B and FIG. 5D, the date correction control 704 may also be configured to select an option 704C indicating that the menstrual period has not started. If the menstrual period has not started, the user may cancel the calibration of the date 2 as the first menstrual day by acting on the correction control 703. The input operation 4 may include some or all of the following: an input operation (for example, a tap operation) performed on the correction control 703, an input operation (for example, a slide operation of selecting the option 704C) performed on the date correction control 704, and an operation (for example, a tap operation) performed on the confirmation control 705.

In some embodiments, with reference to FIG. 5E, along with the first-day prompt information 701, a correction control 707 indicating that the menstrual period does not start is further displayed. The input operation 4 may include an input operation (for example, a tap operation) performed on the correction control 707.

S112: When the current date is a date 3, and the menstrual period trend value 3 meets a preset condition 3, predict and calibrate the date 3 as a last menstrual day of the current menstrual period (menstrual period 1).

S113: Output last-day prompt information.

In this embodiment of this application, it is determined, based on the menstrual period trend value of the current date, that the current day is the last menstrual day, and the user is reminded by using the last-day prompt information. For example, with reference to FIG. 6A, the electronic device 200 indicates the smart band 100 to display last-day prompt information 801. In some embodiments, with reference to FIG. 3H, the user may enable the last day reminder of the menstrual period by using the switch control 503.

In some embodiments, with reference to S107 in FIG. 7B-1 and FIG. 7B-2, the preset condition 2 includes that the menstrual period trend value of the current date (for example, the date 2) is greater than a last-day threshold (for example, 0.8), and the menstrual period trend value continuously decreases for u (for example, 3) days on the current date and a previous date.

In some embodiments, with reference to S107 in FIG. 7B-1 and FIG. 7B-2, the preset condition 2 includes that the menstrual period trend value continuously increases for w days and then decreases for u (for example, 3) days on the current date and a previous date, and the menstrual period trend value of the current date (for example, the date 2) in the u days is less than the last-day threshold (for example, 0.8) for the first time.

In some embodiments, with reference to (b) in FIG. 13, time domain alignment is performed between the sequence 5 corresponding to the menstrual period trend value of the last C days and the historical menstrual period trend sequence. The preset condition 1 includes: after the alignment, a point that is corresponding to the current date (that is, the date 3) in the sequence 5 and that is in the historical menstrual period trend sequence is a point corresponding to the last menstrual day.

S114: When an input operation 5 is detected, determine that the calibrated last menstrual day (that is, the date 3) is correct.

In some embodiments, with reference to FIG. 6B and FIG. 6C, a confirmation control 802 is further displayed along with the predicted last-day prompt information 801. The input operation 5 includes an operation (for example, a tap operation) performed on the confirmation control 802. It may be understood that, if the user determines that the date 2 is the last menstrual day of the menstrual period 1, the input operation 5 acting on the confirmation control 802 may indicate that the prompted last menstrual day is correct.

S115: When an input operation 6 is detected, perform correction and calibrate a date 6 as the last menstrual day of the menstrual period 1.

In some embodiments, with reference to FIG. 6B and FIG. 6C, a correction control 803 is further displayed along with the last-day prompt information 801. If the menstrual period has arrived but it is detected that the last menstrual day is incorrect, the user may correct the date of the last menstrual day by acting on the correction control 803. The input operation 6 may include some or all of the following: an input operation (for example, a tap operation) performed on the correction control 803, an input operation (for example, a slide operation for selecting a date 6) performed on the date correction control 804, and an operation (for example, a tap operation) performed on the confirmation control 805. The date correction control 804 is configured to select the corrected last menstrual day (that is, the date 6), and the confirmation control 805 is configured to confirm the selected date 6.

S116: When an input operation 7 is detected, cancel calibrating the date 3 as the last menstrual day.

In some embodiments, with reference to FIG. 6B and FIG. 6D, the date correction control 804 may also be configured to select an option 804C indicating that the menstrual period does not end. If the menstrual period has not ended, the user can cancel the calibration of the date 3 as the last menstrual day by using the option 804C. The input operation 7 may include some or all of the following: an input operation (for example, a tap operation) performed on the correction control 803, an input operation (for example, a slide operation for selecting the option 804C) performed on the option 804C in the date correction control 804, and an operation (for example, a tap operation) performed on the confirmation control 805.

In some embodiments, with reference to FIG. 6E, along with the last-day prompt information 801, a correction control 807 indicating that the menstrual period does not end is further displayed, and the input operation 7 may include an input operation (for example, a tap operation) performed on the correction control 807.

S117: Determine whether the user confirms the calibrated first day and last day of the menstrual period 1; and if the user confirms the calibrated first day and last day, perform S119; or if the user does not confirm the calibrated first day and last day, perform S118.

If the electronic device 200 has calibrated the first day and last day of the menstrual period 1, and the user has confirmed the calibrated first day and last day of the menstrual period 1, S 119 is performed; otherwise, S118 is performed.

S118: On an F^{th} day after the last menstrual day, perform intelligent re-calibration on the menstrual period 1 based on historical trend values of last X days, where X is greater than F.

In some embodiments, after the menstrual period 1 ends, if the first day and/or the last day of the menstrual period 1 are/is missing (that is, not calibrated), or the first day and the last day of the menstrual period 1 are preliminarily calibrated, but the user does not feed back/confirm the calibrated first day and/or last day of the menstrual period, or the calibrated first day and/or last day of the menstrual period 1 are/is clearly incorrect (for example, the first day and the last day of the menstrual period 1 are the same day), based on menstrual period trend values of F days after the menstrual period ends, the first day and the last day of the menstrual period 1 are intelligently re-calibrated. Using the first menstrual day as an example, that the user has fed back/confirmed the calibrated first menstrual day may mean that the user has manually corrected the first menstrual day (for example, corrected the first menstrual day by using the correction control 703), or has viewed the calibrated first menstrual day (for example, confirmed, by using the confirmation control 702, that the calibrated first menstrual day has been viewed). Accuracy of re-calibrating the menstrual period based on the menstrual period trend values of the F days after the menstrual period ends is higher. In an implementation, on the F^{th} day after the menstrual period ends, a sequence 6 corresponding to the menstrual period trend values of the last X days is obtained, and time domain alignment is performed between the sequence 6 and the historical menstrual period trend sequence. After the alignment, a point that is corresponding to the first menstrual day in the historical menstrual period trend sequence and that is in the sequence 6 is obtained, and a date corresponding to the point in the sequence 6 is re-calibrated as the first menstrual day. A point that is corresponding to the last menstrual day in the historical menstrual period trend sequence and that is in the sequence 6 is obtained, and a date corresponding to the point in the sequence 6 is re-calibrated as the last menstrual day. Optionally, X is equal to Pl.

In some embodiments, after the electronic device 200 preliminarily calibrates the menstrual period, if the user does not perform feedback for correction, the calibrated first menstrual day and last menstrual day are indicated in the calendar of the physiological cycle by using an indicator 1 (for example, a dashed-line box); or if the user feeds back that the preliminarily calibrated menstrual period is confirmed/corrected, the calibrated first menstrual day and last menstrual day are indicated in the calendar of the physiological cycle by using an indicator 2 (for example, a solid-line box).

In some embodiments, after intelligent re-calibration, if the preliminarily calibrated menstrual period is modified, prompt information is output. The prompt information is used to prompt the user that the calibrated menstrual period is corrected, and prompt the user to view the corrected menstrual period. A time (that is, the foregoing F) of intelligent re-calibration may be preset by the user/electronic device 200.

S119: Generate a menstrual period report based on the calibrated menstrual period 1.

After step S117 or S118 (that is, intelligent re-calibration), a physiological evaluation report is generated based on some or all of a menstrual period trend of a last physiological cycle, a physiological feature of the last physiological cycle, a historical menstrual period trend sequence, and a historical physiological feature sequence. The health evaluation report may indicate one or more of the following menstrual period information: a menstrual period length, a menstrual period regularity evaluation, a health evaluation, and the like.

This application provides a menstrual period management method, applied to a first electronic device, and including: when an X^{th} day after a first date is predicted as a first menstrual day of a first menstrual period, displaying first prompt information and a first correction control, where the first prompt information is used to remind a user that the menstrual period arrives within X days, and the first correction control indicates that the first menstrual day of the first menstrual period has arrived, and is further used to correct the first menstrual day of the first menstrual period; and if a first input operation is detected, correcting and calibrating the first menstrual day of the first menstrual period to a second date, where the first input operation includes a second input operation (for example, a tap operation) acting on the first correction control, the second date is earlier than or equal to the first date, and the calibrated first menstrual day is used to predict a future menstrual period.

In an implementation, a date selection control is displayed in response to the second input operation acting on the first correction control, where the date selection control is configured to select a corrected date, the date selection control includes a date option of the second date, and the first input operation further includes a third input operation acting on the date option of the second date.

In an implementation, a first confirmation control is displayed after the third input operation acting on the date option of the second date is detected, and the first input operation further includes a fourth input operation (for example, a tap operation) acting on the first confirmation control.

The first electronic device may include the foregoing electronic device 200, or may include the foregoing smart band 100. The first prompt information may include the foregoing advance prompt information. The first date may include the foregoing date c or date 1. The second date may include the foregoing date 4. The first menstrual period may include the foregoing menstrual period 1. The first input operation may include the foregoing input operation 1. With reference to related descriptions in FIG. 4A to FIG. 4C, the first prompt information may include the advance prompt information 601, the first correction control may include the correction control 603, the date selection control may include the date correction control 604, and the first confirmation control may include the confirmation control 605.

In an implementation, when it is detected that a third date is the first menstrual day, the third date is calibrated as the first menstrual day of the first menstrual period, and second prompt information and a second correction control are displayed, where the second prompt information indicates that the third date is the first menstrual day, and the second correction control is used to correct the first menstrual day of the first menstrual period.

In an implementation, after the second prompt information and the second correction control are displayed, the method further includes: if a fifth input operation is detected, correcting and calibrating the first menstrual day of the first menstrual period to a fourth date, where the fifth input operation includes a sixth input operation (for example, a tap operation) acting on the second correction control.

In an implementation, the method further includes: displaying a date option of the fourth date in response to the sixth input operation, where the fifth input operation further includes an operation acting on the date option of the fourth date.

In an implementation, the method further includes: further displaying a first cancellation option in response to the sixth input operation, where the first cancellation option indicates that the first menstrual day of the first menstrual period has not arrived; and if a seventh input operation acting on the first cancellation option is detected, canceling calibrating the third date as the first menstrual day of the first menstrual period.

In an implementation, the method further includes: further displaying a first cancellation control along with the second prompt information, where the first cancellation control indicates that the first menstrual day of the first menstrual period has not arrived, and is used to cancel calibrating the third date as the first menstrual day of the first menstrual period.

The second prompt information may include the foregoing first-day prompt information, the third date may include the foregoing date c or date 2, the fourth date may include the foregoing date 5, the fifth input operation may include the foregoing input operation 3, and the seventh input operation may include the foregoing input operation 4. With reference to related descriptions in FIG. 5A to FIG. 5E, the second prompt information may include the first-day prompt information 701, the second correction control may include the correction control 703, the first cancellation option may include the option 704C, and the first cancellation control may include the correction control 707.

In an implementation, the method further includes: when it is detected that a fifth date is a last menstrual day, calibrating the fifth date as the last menstrual day of the first menstrual period, and displaying third prompt information and a third correction control, where the third prompt information indicates that the fifth date is the last menstrual day of the first menstrual period, the third correction control is used to correct the last menstrual day of the first menstrual period, and the calibrated last menstrual day is used to predict the future menstrual period.

In an implementation, after the displaying third prompt information and a third correction control, the method further includes: if an eighth input operation is detected, correcting and calibrating the last menstrual day of the first menstrual period to a sixth date, where the eighth input operation includes a ninth input operation acting on the third correction control.

In an implementation, the method further includes: displaying a date option of the sixth date in response to the ninth input operation, where the eighth input operation further includes an operation acting on the date option of the sixth date.

In an implementation, the method further includes: further displaying a second cancellation option in response to the ninth input operation, where the second cancellation option indicates that the first menstrual period has not ended; and if a tenth input operation acting on the second cancellation option is detected, canceling calibrating the fifth date as the last menstrual day of the first menstrual period.

In an implementation, the method further includes: further displaying a second cancellation control along with the third prompt information, where the second cancellation control indicates that the first menstrual period has not ended, and is used to cancel calibrating the fifth date as the last menstrual day of the first menstrual period.

The third prompt information may include the foregoing last-day prompt information, the fifth date may include the foregoing date c or date 3, the sixth date may include the foregoing date 6, the eighth input operation may include the foregoing input operation 6, and the tenth input operation may include the foregoing input operation 7. With reference to related descriptions in FIG. 5A to FIG. 5E, the third prompt information may include the last-day prompt information 801, the third correction control may include the correction control 803, the second cancellation option may include the option 804C, and the second cancellation control may include the control 807.

In an implementation, the first electronic device is further configured to obtain K types of physiological features of the user on every day based on data collected by a detection apparatus, and the method further includes: detecting a menstrual period trend value of a current date based on K types of physiological features of the user on last N days, where the menstrual period trend value of the current date indicates a degree to which the current date is close to a peak of a menstrual period of the user, N and K are positive integers, and the menstrual period trend value is used to predict and calibrate a first menstrual day and a last menstrual day.

In an implementation, the method further includes: updating and displaying a menstrual period trend curve and a first indication box based on the menstrual period trend value of the current date, where the menstrual period trend curve sequentially indicates a detected menstrual period trend value of a date before the current date, the menstrual period trend value of the current date, and a predicted menstrual period trend value of a date after the current date, the first indication box indicates a menstrual period trend value on every day within a menstrual period range on the menstrual period trend curve, and the menstrual period range is a time range from the first menstrual day to the last menstrual day.

With reference to related descriptions in FIG. 3E, the menstrual period trend curve may include the foregoing menstrual period trend curve 301, and the first indication box may include the menstrual period indicator 306.

In an implementation, a menstrual period trend curve corresponding to a date after the current date in the menstrual period trend curve is determined based on a historical menstrual period trend curve/historical menstrual period trend sequence obtained through comprehensive statistics collection; the historical menstrual period trend sequence is determined through comprehensive statistics collection based on a detected menstrual period trend value in at least one historical physiological cycle; the historical menstrual period trend sequence indicates an average length of historical physiological cycles obtained through comprehensive statistics collection and a menstrual period trend value on every day in one historical physiological cycle obtained through comprehensive statistics collection; the historical menstrual period trend curve is determined based on the historical menstrual period trend sequence; and the historical menstrual period trend sequence further indicates a menstrual period range in one physiological cycle corresponding to the historical menstrual period trend sequence, and the menstrual period range indicated by the historical menstrual period trend sequence is determined based on a menstrual period calibrated in the at least one historical physiological cycle.

In an implementation, the method further includes: displaying a first indicator on the menstrual period trend curve, where the first indicator indicates a point corresponding to the current date on the menstrual period trend curve; and after an eleventh input operation (for example, a tap operation) acting on the first indicator is detected, displaying a first interface, where the first interface is used to edit and display a physical symptom corresponding to the current date.

With reference to related descriptions in FIG. 3E, the first indicator may include the indicator 301A, and the first interface may include the user interface 16.

In an implementation, the K types of physiological features include a first physiological feature, and the method further includes: further displaying one or more of the following physiological feature curves of the first physiological feature along with the menstrual period trend curve: a measured physiological feature curve, a historical minimum physiological feature curve, a historical average physiological feature curve, and a historical average physiological feature curve, where the menstrual period trend curve has a same horizontal coordinate as each physiological feature curve, and the measured physiological feature curve and each historical physiological feature curve are aligned in time domain by using a preset algorithm; the measured physiological feature curve indicates physiological feature values measured on the current date and a previous date; the historical minimum physiological feature curve indicates a minimum physiological feature value on every day in one historical physiological cycle obtained through comprehensive statistics collection; the historical average physiological feature curve indicates an average physiological feature value on every day in one historical physiological cycle obtained through comprehensive statistics collection; and the historical maximum physiological feature curve indicates a maximum physiological feature value on every day in one historical physiological cycle obtained through comprehensive statistics collection.

In an implementation, the method further includes: detecting, based on a detected menstrual period trend value of the user, whether an abnormal situation exists; and displaying fourth prompt information when an abnormal situation is detected, where the fourth prompt information indicates the abnormal situation.

The abnormal situation includes any one of the foregoing abnormal situation 1 to abnormal situation 4. When the abnormal situation is the abnormal situation 1, the fourth prompt information may include the advance prompt information 3. When the abnormal situation is the abnormal situation 3, the fourth prompt information may include the advance prompt information 4. When the abnormal situation is the abnormal situation 4, the fourth prompt information may include the advance prompt information 5.

In an implementation, the abnormal situation includes one or more of the following: the user is changed; the user is not a female; the user is sick; or the user is pregnant; and when the abnormal situation is that the user is not a female, the fourth prompt information is further used to prompt the user to disable a related function for a menstrual period; or when the abnormal situation is that the user is pregnant, the fourth prompt information is further used to prompt the user to switch to a pregnancy mode.

In an implementation, the method further includes: on an F^{th} day after a last menstrual day of the first menstrual period, re-calibrating the first menstrual day and the last menstrual day of the first menstrual period based on detected menstrual period trend values on last C days and a historical menstrual period trend sequence, where C is greater than F, and both C and F are positive integers.

In an implementation, the re-calibrating the first menstrual day and the last menstrual day of the first menstrual period includes: when a first condition is detected, re-calibrating the first menstrual day and the last menstrual day of the first menstrual period, where the first condition includes one or more of the following: the first menstrual day and/or the last menstrual day of the first menstrual period are/is not calibrated; the first menstrual day and/or the last menstrual day are/is calibrated, but the user does not provide a feedback for the calibrated first menstrual day and/or last menstrual day; or the calibrated first menstrual day and/or last menstrual day are/is clearly incorrect.

In an implementation, when the menstrual period trend value of the current date meets a second condition, an X^{th} day after the current date is predicted as the first menstrual day of the first menstrual period, where the second condition includes: a difference between the menstrual period trend value of the current date and a first threshold is less than a preset value, and the first threshold is a menstrual period trend value of an X^{th} day before the first menstrual day that is determined based on a historical menstrual period trend sequence.

In an implementation, when the menstrual period trend value of the current date meets a third condition, the current date is predicted as the first menstrual day of the first menstrual period, where the third condition includes: on the current date and a previous date, the menstrual period trend value continuously increases by w days, and the menstrual period trend value of the current date is greater than a first-day threshold for the first time within the w days.

In an implementation, when the menstrual period trend value of the current date meets a fourth condition, the current date is predicted as a last menstrual day of the first menstrual period, where the fourth condition includes: on the current date and a previous date, the menstrual period trend value continuously increases by w days and then continuously decreases by u days, and the menstrual period trend value of the current date is less than a last-day threshold for the first time within the u days.

The second condition may include the foregoing preset condition 1, the third condition may include the foregoing preset condition 2, the fourth condition may include the foregoing preset condition 3, the first threshold may include the foregoing threshold 1, and the preset value may include the foregoing preset value 1.

Implementations of this application may be randomly combined to achieve different technical effects.

All or some of the foregoing embodiments may be implemented by software, hardware, firmware, or any combination thereof. When software is used to implement the embodiments, all or some of the embodiments may be implemented in a form of a computer program product. The computer program product includes one or more computer instructions. When the computer program instructions are loaded and executed on a computer, the procedures or functions according to this application are all or partially generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

A person of ordinary skill in the art may understand that all or some of the procedures in the methods in the foregoing embodiments may be completed by a computer program instructing related hardware. The program may be stored in a computer-readable storage medium. When the program is executed, the procedures in the foregoing method embodiments may be included. The foregoing storage medium includes any medium that can store program code, such as a ROM or a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, or improvement made according to the disclosure of the present invention shall fall within the protection scope of the present invention.

## Claims

1. A menstrual period management method, applied to a first electronic device, and comprising:
when an X^{th} day after a first date is predicted as a first menstrual day of the first menstrual period, displaying first prompt information and a first correction control, wherein the first prompt information is used to remind a user that the menstrual period arrives within X days, and the first correction control indicates that the first menstrual day of the first menstrual period has arrived, and is further used to correct the first menstrual day of the first menstrual period; and
if a first input operation is detected, correcting and calibrating the first menstrual day of the first menstrual period to a second date, wherein the first input operation comprises a second input operation acting on the first correction control, the second date is earlier than or equal to the first date, and the calibrated first menstrual day is used to predict a future menstrual period.

2. The method according to claim 1, further comprising:
displaying a date selection control in response to the second input operation acting on the first correction control, wherein the date selection control is configured to select a corrected date, the date selection control comprises a date option of the second date, and the first input operation further comprises a third input operation acting on the date option of the second date.

3. The method according to claim 2, wherein a first confirmation control is displayed after the third input operation acting on the date option of the second date is detected, and the first input operation further comprises a fourth input operation acting on the first confirmation control.

4. The method according to claim 1, further comprising:
when it is detected that a third date is the first menstrual day, calibrating the third date as the first menstrual day of the first menstrual period, and displaying second prompt information and a second correction control, wherein the second prompt information indicates that the third date is the first menstrual day, and the second correction control is used to correct the first menstrual day of the first menstrual period.

5. The method according to claim 4, wherein after the displaying second prompt information and a second correction control, the method further comprises:
if a fifth input operation is detected, correcting and calibrating the first menstrual day of the first menstrual period to a fourth date, wherein the fifth input operation comprises a sixth input operation acting on the second correction control.

6. The method according to claim 5, further comprising:
displaying a date option of the fourth date in response to the sixth input operation, wherein the fifth input operation further comprises an operation acting on the date option of the fourth date.

7. The method according to claim 6, further comprising:
further displaying a first cancellation option in response to the sixth input operation, wherein the first cancellation option indicates that the first menstrual day of the first menstrual period has not arrived; and
if a seventh input operation acting on the first cancellation option is detected, canceling calibrating the third date as the first menstrual day of the first menstrual period.

8. The method according to claim 4, further comprising:
further displaying a first cancellation control along with the second prompt information, wherein the first cancellation control indicates that the first menstrual day of the first menstrual period has not arrived, and is used to cancel calibrating the third date as the first menstrual day of the first menstrual period.

9. The method according to claim 1, further comprising:
when it is detected that a fifth date is a last menstrual day, calibrating the fifth date as the last menstrual day of the first menstrual period, and displaying third prompt information and a third correction control, wherein the third prompt information indicates that the fifth date is the last menstrual day of the first menstrual period, the third correction control is used to correct the last menstrual day of the first menstrual period, and the calibrated last menstrual day is used to predict the future menstrual period.

10. The method according to claim 9, wherein after the displaying third prompt information and a third correction control, the method further comprises:
if an eighth input operation is detected, correcting and calibrating the last menstrual day of the first menstrual period to a sixth date, wherein the eighth input operation comprises a ninth input operation acting on the third correction control.

11. The method according to claim 10, further comprising:
displaying a date option of the sixth date in response to the ninth input operation, wherein the eighth input operation further comprises an operation acting on the date option of the sixth date.

12. The method according to claim 10, further comprising:
further displaying a second cancellation option in response to the ninth input operation, wherein the second cancellation option indicates that the first menstrual period has not ended; and
if a tenth input operation acting on the second cancellation option is detected, canceling calibrating the fifth date as the last menstrual day of the first menstrual period.

13. The method according to claim 4, further comprising:
further displaying a second cancellation control along with the third prompt information, wherein the second cancellation control indicates that the first menstrual period has not ended, and is used to cancel calibrating the fifth date as the last menstrual day of the first menstrual period.

14. The method according to claim 1, wherein the first electronic device is further configured to obtain K types of physiological features of the user on every day based on data collected by a detection apparatus, and the method further comprises:
detecting a menstrual period trend value of a current date based on K types of physiological features of the user on last N days, wherein the menstrual period trend value of the current date indicates a degree to which the current date is close to a peak of a menstrual period of the user, N and K are positive integers, and the menstrual period trend value is used to predict and calibrate a first menstrual day and a last menstrual day.

15. The method according to claim 14, further comprising:
updating and displaying the menstrual period trend curve and a first indication box based on the menstrual period trend value of the current date, wherein the menstrual period trend curve sequentially indicates a detected menstrual period trend value of a date before the current date, the menstrual period trend value of the current date, and a predicted menstrual period trend value of a date after the current date, the first indication box indicates a menstrual period trend value on every day within a menstrual period range on the menstrual period trend curve, and the menstrual period range is a time range from the first menstrual day to the last menstrual day.

16. The method according to claim 15, wherein a menstrual period trend curve corresponding to a date after the current date in the menstrual period trend curve is determined based on a historical menstrual period trend curve/historical menstrual period trend sequence obtained through comprehensive statistics collection; the historical menstrual period trend sequence is determined through comprehensive statistics collection based on a detected menstrual period trend value in at least one historical physiological cycle; the historical menstrual period trend sequence indicates an average length of historical physiological cycles obtained through comprehensive statistics collection and a menstrual period trend value on every day in one historical physiological cycle obtained through comprehensive statistics collection; the historical menstrual period trend curve is determined based on the historical menstrual period trend sequence; and the historical menstrual period trend sequence further indicates a menstrual period range in one physiological cycle corresponding to the historical menstrual period trend sequence, and the menstrual period range indicated by the historical menstrual period trend sequence is determined based on a menstrual period calibrated in the at least one historical physiological cycle.

17. The method according to claim 15, further comprising:
displaying a first indicator on the menstrual period trend curve, wherein the first indicator indicates a point corresponding to the current date on the menstrual period trend curve; and
after an eleventh input operation acting on the first indicator is detected, displaying a first interface, wherein the first interface is used to edit and display a physical symptom corresponding to the current date.

18. The method according to claim 1 or 15, wherein the K types of physiological features comprise a first physiological feature, and the method further comprises:
further displaying one or more of the following physiological feature curves of the first physiological feature along with the menstrual period trend curve: a measured physiological feature curve, a historical minimum physiological feature curve, a historical average physiological feature curve, and a historical average physiological feature curve, wherein the menstrual period trend curve has a same horizontal coordinate as each physiological feature curve, and the measured physiological feature curve and each historical physiological feature curve are aligned in time domain by using a preset algorithm;
the measured physiological feature curve indicates physiological feature values measured on the current date and a previous date;
the historical minimum physiological feature curve indicates a minimum physiological feature value on every day in one historical physiological cycle obtained through comprehensive statistics collection;
the historical average physiological feature curve indicates an average physiological feature value on every day in one historical physiological cycle obtained through comprehensive statistics collection; and
the historical maximum physiological feature curve indicates a maximum physiological feature value on every day in one historical physiological cycle obtained through comprehensive statistics collection.

19. The method according to claim 1 or 14, further comprising:
detecting, based on a detected menstrual period trend value of the user, whether an abnormal situation exists; and
displaying fourth prompt information when an abnormal situation is detected, wherein the fourth prompt information indicates the abnormal situation.

20. The method according to claim 19, wherein the abnormal situation comprises one or more of the following: the user is changed; the user is not a female; the user is sick; or the user is pregnant; and
when the abnormal situation is that the user is not a female, the fourth prompt information is further used to prompt the user to disable a related function for a menstrual period; or
when the abnormal situation is that the user is pregnant, the fourth prompt information is further used to prompt the user to switch to a pregnancy mode.

21. The method according to claim 1 or 14, further comprising:
on an F^{th} day after a last menstrual day of the first menstrual period, re-calibrating the first menstrual day and the last menstrual day of the first menstrual period based on detected menstrual period trend values on last C days and a historical menstrual period trend sequence, wherein C is greater than F, and both C and F are positive integers.

22. The method according to claim 1 or 14, wherein the re-calibrating the first menstrual day and the last menstrual day of the first menstrual period comprises:
when a first condition is detected, re-calibrating the first menstrual day and the last menstrual day of the first menstrual period, wherein
the first condition comprises one or more of the following: the first menstrual day and/or the last menstrual day of the first menstrual period are/is not calibrated; the first menstrual day and/or the last menstrual day are/is calibrated, but the user does not provide a feedback for the calibrated first menstrual day and/or last menstrual day; or the calibrated first menstrual day and/or last menstrual day are/is clearly incorrect.

23. The method according to claim 14, wherein
when the menstrual period trend value of the current date meets a second condition, an X^{th} day after the current date is predicted as the first menstrual day of the first menstrual period, wherein the second condition comprises: a difference between the menstrual period trend value of the current date and a first threshold is less than a preset value, and the first threshold is a menstrual period trend value of an X^{th} day before the first menstrual day that is determined based on a historical menstrual period trend sequence.

24. The method according to claim 14, wherein
when the menstrual period trend value of the current date meets a third condition, the current date is predicted as the first menstrual day of the first menstrual period, wherein the second condition comprises: on the current date and a previous date, the menstrual period trend value continuously increases by w days, and the menstrual period trend value of the current date is greater than a first-day threshold for the first time within the w days.

25. The method according to claim 14, wherein
when the menstrual period trend value of the current date meets a fourth condition, the current date is predicted as a last menstrual day of the first menstrual period, wherein the fourth condition comprises: on the current date and a previous date, the menstrual period trend value continuously increases by w days and then continuously decreases by u days, and the menstrual period trend value of the current date is less than a last-day threshold for the first time within the u days.

26. A range processing apparatus, comprising a memory, one or more processors, and one or more programs, wherein the one or more programs are stored in the memory; and when the one or more processors execute the one or more programs, the access device is enabled to implement the method according to any one of claims 1 to 25.

27. A computer storage medium, comprising computer instructions, wherein when the computer instructions are run on an electronic device, the terminal is enabled to implement the method according to any one of claims 1 to 25.
